# EUROPEAN PATENT SPECIFICATION

(11) **EP 2 250 163 B1**
(45) Date of publication and mention of the grant of the patent: **28.03.2012**
(21) Application number: 08780411.8
(22) Date of filing: 12.02.2008
(51) Int. Cl.: C07D 403/10, C07D 403/14, A61K 31/4164, A61P 31/22

(54) **HEPATITIS C VIRUS INHIBITORS**
INHIBITOREN DES HEPATITIS-C-VIRUS
INHIBITEURS DU VIRUS DE L'HÉPATITE C

(43) Date of publication of application: 17.11.2010
(73) Proprietor: Bristol-Myers Squibb Company, Princeton, NJ 05843-4000 (US)
(72) Inventor: BACHAND, Carol, Candiac, Québec J5R 1B1 (CA); BELEMA, Makonen, Wallingford, Connecticut 06492 (US); DEON, Daniel, H., Montreal, Québec H4A 2N4 (CA); GOOD, Andrew, C., Wallingford, Connecticut 06492 (US); GOODRICH, Jason, Wallingford, Connecticut 06492 (US); JAMES, Clint, A., Longueuil, Québec J4H 2N4 (CA); LAVOIE, Rico, Candiac, Quebec J5R 4B7 (US); LOPEZ, Omar, D., Wallingford, Connecticut 06492 (US); MARTEL, Alain, Delson, Québec J5B 1G8 (CA); MEANWELL, Nicholas, A., Wallingford, Connecticut 06492 (US); NGUYEN, Van N., Wallingford, Connecticut 06492 (US); ROMINE, Jeffrey, Lee, Wallingford, Connecticut 06492 (US); RUEDIGER, Edward, H., Greenfield Park, Québec J4V 2K3 (CA); SNYDER, Lawrence, B., Wallingford, Connecticut 06492 (US); ST. LAURENT, Denis, R., Wallingford, Connecticut 06492 (US); YANG, Fukang, Wallingford, Connecticut 06492 (US); LANGLEY, David, R., Wallingford, Connecticut 06492 (US); WANG, Gan, Wallingford, Connecticut 06492 (US); HAMANN, Lawrence, G., North Grafton, Massachusetts 01536 (US)
(74) Representative: Reitstötter - Kinzebach
(86) International application number: PCT/US2008/053638
(87) International publication number: WO 2009/102318

(56) References cited:
- WO-A-2006/133326
- WO-A-2008/021927
- WO-A-2008/021928
- WO-A-2008/021936
- WO-A-2008/144380

## Description

The present disclosure is generally directed to antiviral compounds, and more specifically directed to compounds which can inhibit the function of the NS5A protein encoded by Hepatitis C virus (HCV), compositions comprising such compounds, and methods for inhibiting the function of the NS5A protein.

HCV is a major human pathogen, infecting an estimated 170 million persons worldwide - roughly five times the number infected by human immunodeficiency virus type 1. A substantial fraction of these HCV infected individuals develop serious progressive liver disease, including cirrhosis and hepatocellular carcinoma.

Presently, the most effective HCV therapy employs a combination of alpha-interferon and ribavirin, leading to sustained efficacy in 40% of patients. Recent clinical results demonstrate that pegylated alpha-interferon is superior to unmodified alpha-interferon as monotherapy. However, even with experimental therapeutic regimens involving combinations of pegylated alpha-interferon and ribavirin, a substantial fraction of patients do not have a sustained reduction in viral load. Thus, there is a clear and long-felt need to develop effective therapeutics for treatment of HCV infection.

HCV is a positive-stranded RNA virus. Based on a comparison of the deduced amino acid sequence and the extensive similarity in the 5' untranslated region, HCV has been classified as a separate genus in the Flaviviridae family. All members of the Flaviviridae family have enveloped virions that contain a positive stranded RNA genome encoding all known virus-specific proteins via translation of a single, uninterrupted, open reading frame.

Considerable heterogeneity is found within the nucleotide and encoded amino acid sequence throughout the HCV genome. At least six major genotypes have been characterized, and more than 50 subtypes have been described. The major genotypes of HCV differ in their distribution worldwide, and the clinical significance of the genetic heterogeneity of HCV remains elusive despite numerous studies of the possible effect of genotypes on pathogenesis and therapy.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature non-structural proteins (NS2, NS3, NS4A, NS4B, NSSA, and NS5B) is effected by two viral proteases. The first one is believed to be a metalloprotease and cleaves at the NS2-NS3 junction; the second one is a serine protease contained within the N-terminal region of NS3 (also referred to herein as NS3 protease) and mediates all the subsequent cleavages downstream of NS3, both in cis, at the NS3-NS4A cleavage site, and in trans, for the remaining NS4A-NS4B, NS4B-NS5A, NS5A-NS5B sites. The NS4A protein appears to serve multiple functions, acting as a cofactor for the NS3 protease and possibly assisting in the membrane localization of NS3 and other viral replicase components. The complex formation of the NS3 protein with NS4A seems necessary to the processing events, enhancing the proteolytic efficiency at all of the sites. The NS3 protein also exhibits nucleoside triphosphatase and RNA helicase activities. NS5B (also referred to herein as HCV polymerase) is a RNA-dependent RNA polymerase that is involved in the replication of HCV.

Compounds useful for treating HCV-infected patients are desired which selectively inhibit HCV viral replication. In particular, compounds which are effective to inhibit the function of the NS5A protein are desired. The HCV NS5A protein is described, for example, in Tan, S.-L., Katzel, M.G. Virology 2001, 284, 1-12; and in Park, K.-J.; Choi, S.-H, J. Biological Chemistry 2003. WO 2006/133326 describes HCV inhibitor.

In its first aspect the present disclosure provides a compound selected from
dimethyl (4,4'-biphenyldiylbis(1H-imidazole-4,2-diyl((1S)-2-methyl-1,1-propanediyl)imino((2S)-1-oxo-1,2-propanediyl)))biscarbamate
dimethyl (4,4'-biphenyldiylbis(1H-imidazole-4,2-diyl((1S)-2-meth-1,1-propanediyl)imino((2S,3R)-3-methoxy-1-oxo-1,2-butanediyl)))biscarbamate;
dimethyl (4,4'-biphenyldiylbis(1H-imidazole-4,2-diyl((1S)-2-methyl-1,1-propanediyl)imino((2S)-3-methyl-1-oxo-1,2-butanediyl)))biscarbamate:

dimethyl (4,4'-biphenyldiylbis(1H-imidazole-4,2-diyl((1S)-2-methyl-1,1-pronanediyl)imino((2S)-4-methoxy-1-oxo-1,2-butanediyl)))biscarbamate;
dimethyl (4,4'-biphenyldiylbis(1H-imidazole-4,2-diyl((1R)-2-methyl-1,1-propanediyl)imino((2S)-3-methyl-1-oxo-1,2-butanediyl)))biscarbamate;
dimethyl (4,4'-biphenyldiylbis(1H-imidazole-4,2-diyl((1R)-2-methyl-1,1-propanediyl)imino((2S)-1-oxo-1,2-propanediyl)))biscarbamate:
dimethyl (4,4'-biphenyldiylbis(1H-imidazole-4,2-diyl((1R)-2-methyl-1,1-propanediyl)imino((2S)-4-methoxy-1-oxo-1,2-butanediyl)))biscarbamate;
dimethyl (4,4'-biphenyldiylbis(1H-imidazole-4,2-diyl((1R)-2-methyl-1,1-propanediyl)imino((2S,3R)-3-methoxy-1-oxo-1,2-butanediyl)))biscarbamate;
dimethyl (4,4'-biphenyldiylbis(1H-imidazole-4,2-diyl((1R)-2-methyl-1,1-propanediyl)imino((2R)-3-methyl-1-oxo-1,2-butanediyl)))biscarbamate;
dimethyl (4,4'-biophenyldiylbis(1H-imidazole-4,2-diyl((1S)-2-methyl-1,1-propanediyl)imino((2R)-3-methyl-1-oxo-1,2-butanediyl)))biscarbamate;
N²-(methoxycarbonyl)-N-((1S)-1-(4-(4'-(2-((2S)-1-(N-(methoxycarbonyl)-L-valyl)-2-pyrrolidinyl)-1H-imidazol-4-yl)-4-biphenylyl)-1H-imidazol-2-yl)-2-methylpropyl)-L-valinamide:
methyl((1S,2R)-2-methoxy-1-(((2S)-2-(4-(4'-(2-((1S)-1-((N-(methoxycarbonyl)-O-methyl-L-threonyl)amino)-2-methylpropyl)-1H-imidazol-4-yl)-4-biphenylyl)-1H-imidazol-2-yl)-1-pyrrolidinyl)carbonyl)propyl)carbamate;
methyl ((1S)-3-methoxy-1-(((2S)-2-(4-(4'-(2-((1S)-1-((N-(methoxycarbonyl)-O-methyl-L-homoseryl)amino)-2-methylpropyl)-1H-imidazol-4-yl)-4-biphenylyl)-1H-imidazol-2-yl)-1-pyrrolidinyl)carbonyl)propyl)carbamate;
methyl ((1S)-2-((2S)-2-(4-(4'-(2-((1S)-1-((N-(methoxycarbonyl)-L-alanyl)amino)-2-methylpropyl)-1H-imidazol-4-yl)-4-biphenylyl)-1H-imidazol-2-yl)-1-pyrrolidinyl)-1-methyl-2-oxoethyl)carbamate;
N²-(methoxycarbonyl)-N-((1R)-1-(4-(4'-(2-((2S)-1-(N-(methoxycarbonyl)-L-valyl)-2-pyrrolidinyl)-1H-imidazol-4-yl)-4-biphenylyl)-1H-imidazol-2-yl)-2-methylpropyl)-L-valinamide:
methyl ((1S,2R)-2-methoxy-1-(((2S)-2-(4-(4'-(2-((1R)-1-((N-(methoxycarbonyl)-O-methyl-L-threonyl)amino)-2-methylpropyl)-1H-imidazol-4-yl)-4-biphenylyl)-1H-imidazol-2-yl)-1-pyrrolidinyl)carbonyl)propyl)carbamate;
methyl ((1S)-3-methoxy-1-((2S)-2-(4-(4'-(2-((1R)-1-((N-(methoxycarbonyl)-O-methyl-L-homoseryl)amino)-2-methylpropyl)-1H-imidazol-4-yl)-4-biphenylyl)-1H-imidazol-2-yl)-1-pyrrolidinyl)carbonyl)propyl)carbamate; and
methyl((1S)-2-((2S)-2-(4-(4'-(2-((1R)-1-((N-(methoxycarbonyl)-L-alanyl)amino)-2-methylpropyl)-1H-imidazol-4-yl)4-biphenylyl)-1H-imidazol-2-yl)-1-pyrrolidinyl)-1-methyl-2-oxoethyl)carbamate;
or a pharmaceutically acceptable salt thereof.

In a second aspect the present disclosure provides a compound of (II) selected from 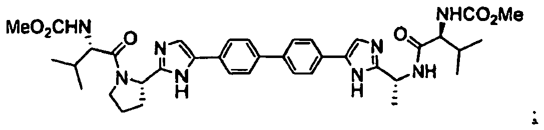 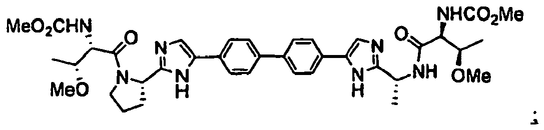 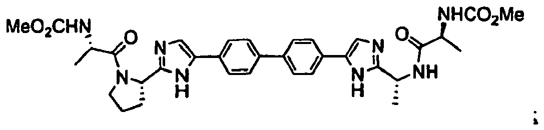 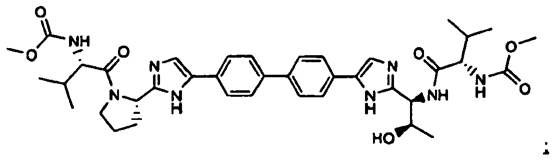 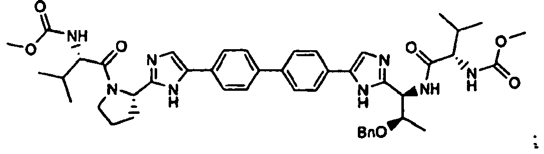 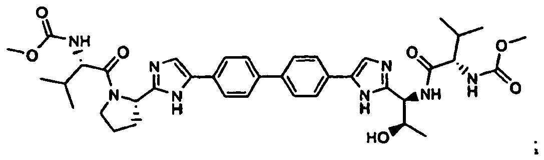 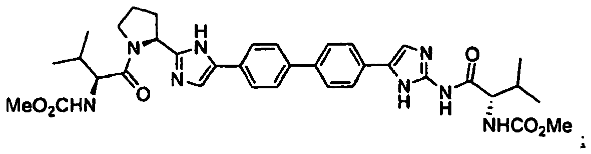 or a pharmaceutically acceptable salt thereof.

In a third aspect the present disclosure provides a composition comprising a compound of the invention, or a pharmaceutically acceptable salt thereof, and a pharmaceutically acceptable carrier. In a first embodiment of the third aspect, the composition comprises one or two additional compounds having anti-HCV activity. In a second embodiment of the third aspect at least one of the additional compounds is an interferon or a ribavirin. In a third embodiment of the third aspect the interferon is selected from interferon alpha 2B, pegylated interferon alpha, consensus interferon, interferon alpha 2A, and lymphoblastiod interferon tau.

In a fourth embodiment of the third aspect the present disclosure provides a composition comprising a compound of the invention, or a pharmaceutically acceptable salt thereof, a pharmaceutically acceptable carrier, and one or two additional compounds having anti-HCV activity, wherein at least one of the additional compounds is selected from interleukin 2, interleukin 6, interleukin 12, a compound that enhances the development of a type I helper T cell response, interfering RNA, anti-sense RNA, Imiqimod, ribavirin, an inosine 5'-monophospate dehydrogenase inhibitor, amantadine, and rimantadine.

In a fifth embodiment of the third aspect the present disclosure provides a composition comprising a compound of the invention, or a pharmaceutically acceptable salt thereof, a pharmaceutically acceptable carrier, and one or two additional compounds having anti-HCV activity, wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection.

In a fourth aspect the present disclosure provides a compound of the invention for use in a method of treating an HCV infection in a patient, comprising administering to the patient a therapeutically effective amount of a compound of the invention, or a pharmaceutically acceptable salt thereof. In a first embodiment of the fourth aspect the method further comprises administering one or two additional compounds having anti-HCV activity prior to, after or simultaneously with the compound of the invention, or a pharmaceutically acceptable salt thereof. In a second embodiment of the fourth aspect at least one of the additional compounds is an interferon or a ribavirin. In a third embodiment of the fourth aspect the interferon is selected from interferon alpha 2B, pegylated interferon alpha, consensus interferon, interferon alpha 2A, and lymphoblastiod interferon tau.

In a fourth embodiment of the fourth aspect the present disclosure provides a compound of the invention for use in a method of treating an HCV infection in a patient, comprising administering to the patient a therapeutically effective amount of a compound of the invention, or a pharmaceutically acceptable salt thereof and adminstering one or two additional compounds having anti-HCV activity prior to, after or simultaneously with the compound of the invention, or a pharmaceutically acceptable salt thereof, wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

In a fifth embodiment of the fourth aspect the present disclosure provides a compound of the invention for use in a method of treating an HCV infection in a patient, comprising administering to the patient a therapeutically effective amount of a compound of the invention, or a pharmaceutically acceptable salt thereof and adminstering one or two additional compounds having anti-HCV activity prior to, after or simultaneously with the compound of the invention, or a pharmaceutically acceptable salt thereof, wherein at least one of the additional compounds is effective to inhibit the function of a target selected from HCV metalloprotease, HCV serine protease, HCV polymerase, HCV helicase, HCV NS4B portein, HCV entry, HCV assembly, HCV egress, HCV NS5A protein, and IMPDH for the treatment of an HCV infection.

Other embodiments of the present disclosure may comprise suitable combinations of two or more of embodiments and/or aspects disclosed herein.

Yet other embodiments and aspects of the disclosure will be apparent according to the description provided below.

The compounds of the present disclosure also exist as tautomers; therefore the present disclosure also encompasses all tautomeric forms.

The description of the present disclosure herein should be construed in congruity with the laws and principals of chemical bonding. In some instances it may be necessary to remove a hydrogen atom in order accommodate a substitutent at any given location. For example, in the structure shown below 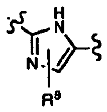 R⁸ may be attached to either the carbon atom in the imidazole ring or, alternatively, R⁸ may take the place of the hydrogen atom on the nitrogen ring to form an N-substituted imidazole.

It should be understood that the compounds encompassed by the present disclosure are those that are suitably stable for use as pharmaceutical agent.

All patents, patent applications, and literature references cited in the specification are herein incorporated by reference in their entirety. In the case of inconsistencies, the present disclosure, including definitions, will prevail.

As used in the present specification, the following terms have the meanings indicated:

As used herein, the singular forms "a", "an", and "the" include plural reference unless the context clearly dictates otherwise.

Asymmetric centers exist in the compounds of the present disclosure. These centers are designated by the symbols "R" or "S", depending on the configuration of substituents around the chiral carbon atom. It should be understood that the disclosure encompasses all stereochemical isomeric forms, or mixtures thereof, which possess the ability to inhibit NS5A. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, or direct separation of enantiomers on chiral chromatographic columns. Starting compounds of particular stereochemistry are either commercially available or can be made and resolved by techniques known in the art.

Certain compounds of the present disclosure may also exist in different stable conformational forms which may be separable. Torsional asymmetry due to restricted rotation about an asymmetric single bond, for example because of steric hindrance or ring strain, may permit separation of different conformers. The present disclosure includes each conformational isomer of these compounds and mixtures thereof.

The term "compounds of the present disclosure", and equivalent expressions, are meant to embrace compounds of the invention, and pharmaceutically acceptable enantiomers, diastereomers, and salts thereof. Similarly, references to intermediates are meant to embrace their salts where the context so permits.

The compounds of the present disclosure can exist as pharmaceutically acceptable salts. The term "pharmaceutically acceptable salt," as used herein, represents salts or zwitterionic forms of the compounds of the present disclosure which are water or oil-soluble or dispersible, which are, within the scope of sound medical judgment, suitable for use in contact with the tissues of patients without excessive toxicity, irritation, allergic response, or other problem or complication commensurate with a reasonable benefit/risk ratio, and are effective for their intended use The salts can be prepared during the final isolation and purification of the compounds or separately by reacting a suitable nitrogen atom with a suitable acid. Representative acid addition salts include acetate, adipate, alginate, citrate, aspartate, benzoate, benzenesulfonate, bisulfate, butyrate, camphorate, camphorsulfonate; digluconate, dihydrobromide, diydrochloride, dihydroiodide, glycerophosphate, hemisulfate, heptanoate, hexanoate, formate, fumarate, hydrochloride, hydrobromide, hydroiodide, 2-hydroxyethanesulfonate, lactate, maleate, mesitylenesulfonate, methanesulfonate, naphthylenesulfonate, nicotinate, 2-naphthalenesulfonate, oxalate, palmoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Examples of acids which can be employed to form pharmaceutically acceptable addition salts include inorganic acids such as hydrochloric, hydrobromic, sulfuric, and phosphoric, and organic acids such as oxalic, maleic, succinic, and citric.

Basic addition salts can be prepared during the final isolation and purification of the compounds by reacting a carboxy group with a suitable base such as the hydroxide, carbonate, or bicarbonate of a metal cation or with ammonia or an organic primary, secondary, or tertiary amine. The cations of pharmaceutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, and N,N'-dibenzylethylenediamine. Other representative organic amines useful for the formation of base addition salts include ethylenediamine, ethanolamine, diethanolamine, piperidine, and piperazine.

When it is possible that for use in therapy, therapeutically effective amounts of a compound of the invention, as well as pharmaceutically acceptable salts thereof, may be administered as the raw chemical, it is possible to present the active ingredient as a pharmaceutical composition. Accordingly, the disclosure further provides pharmaceutical compositions which include therapeutically effective amounts of compounds of the invention or pharmaceutically acceptable salts thereof, and one or more pharmaceutically acceptable carriers, diluents, or excipients. The term "therapeutically effective amount," as used herein, refers to the total amount of each active component that is sufficient to show a meaningful patient benefit, e.g., a reduction in viral load. When applied to an individual active ingredient, administered alone, the term refers to that ingredient alone. When applied to a combination, the term refers to combined amounts of the active ingredients that result in the therapeutic effect, whether administered in combination, serially, or simultaneously. The compounds of the invention and pharmaceutically acceptable salts thereof, are as described above. The carrier(s), diluent(s), or excipient(s) must be acceptable in the sense of being compatible with the other ingredients of the formulation and not deleterious to the recipient thereof. In accordance with another aspect of the present disclosure there is also provided a process for the preparation of a pharmaceutical formulation including admixing a compound of the invention, or a pharmaceutically acceptable salt thereof, with one or more pharmaceutically acceptable carriers, diluents, or excipients. The term "pharmaceutically acceptable," as used herein, refers to those compounds, materials, compositions, and/or dosage forms which are, within the scope of sound medical judgment, suitable for use in contact with the tissues of patients without excessive toxicity, irritation, allergic response, or other problem or complication commensurate with a reasonable benefit/risk ratio, and are effective for their intended use.

Pharmaceutical formulations may be presented in unit dose forms containing a predetermined amount of active ingredient per unit dose. Dosage levels of between about 0.01 and about 250 milligram per kilogram ("mg/kg") body weight per day, preferably between about 0.05 and about 100 mg/kg body weight per day of the compounds of the present disclosure are typical in a monotherapy for the prevention and treatment of HCV mediated disease. Typically, the pharmaceutical compositions of this disclosure will be administered from about 1 to about 5 times per day or alternatively, as a continuous infusion. Such administration can be used as a chronic or acute therapy. The amount of active ingredient that may be combined with the carrier materials to produce a single dosage form will vary depending on the condition being treated, the severity of the condition, the time of administration, the route of administration, the rate of excretion of the compound employed, the duration of treatment, and the age, gender, weight, and condition of the patient. Preferred unit dosage formulations are those containing a daily dose or sub-dose, as herein above recited, or an appropriate fraction thereof, of an active ingredient. Treatment may be initiated with small dosages substantially less than the optimum dose of the compound. Thereafter, the dosage is increased by small increments until the optimum effect under the circumstances is reached. In general, the compound is most desirably administered at a concentration level that will generally afford antivirally effective results without causing any harmful or deleterious side effects.

When the compositions of this disclosure comprise a combination of a compound of the present disclosure and one or more additional therapeutic or prophylactic agent, both the compound and the additional agent are usually present at dosage levels of between about 10 to 150%, and more preferably between about 10 and 80% of the dosage normally administered in a monotherapy regimen.

Pharmaceutical formulations may be adapted for administration by any appropriate route, for example by the oral (including buccal or sublingual), rectal, nasal, topical (including buccal, sublingual, or transdermal), vaginal, or parenteral (including subcutaneous, intracutaneous, intramuscular, intra-articular, intrasynovial, intrasternal, intrathecal, intralesional, intravenous, or intradermal injections or infusions) route. Such formulations may be prepared by any method known in the art of pharmacy, for example by bringing into association the active ingredient with the carrier(s) or excipient(s). Oral administration or administration by injection are preferred.

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil emulsions.

For instance, for oral administration in the form of a tablet or capsule, the active drug component can be combined with an oral, non-toxic pharmaceutically acceptable inert carrier such as ethanol, glycerol, water, and the like. Powders are prepared by comminuting the compound to a suitable fine size and mixing with a similarly comminuted pharmaceutical carrier such as an edible carbohydrate, as, for example, starch or mannitol. Flavoring, preservative, dispersing, and coloring agent can also be present.

Capsules are made by preparing a powder mixture, as described above, and filling formed gelatin sheaths. Glidants and lubricants such as colloidal silica, talc, magnesium stearate, calcium stearate, or solid polyethylene glycol can be added to the powder mixture before the filling operation. A disintegrating or solubilizing agent such as agar-agar, calcium carbonate, or sodium carbonate can also be added to improve the availability of the medicament when the capsule is ingested.

Moreover, when desired or necessary, suitable binders, lubricants, disintegrating agents, and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, and the like. Lubricants used in these dosage forms include sodium oleate, sodium chloride, and the like. Disintegrators include, without limitation, starch, methyl cellulose, agar, betonite, xanthan gum, and the like. Tablets are formulated, for example, by preparing a powder mixture, granulating or slugging, adding a lubricant and disintegrant, and pressing into tablets. A powder mixture is prepared by mixing the compound, suitable comminuted, with a diluent or base as described above, and optionally, with a binder such as carboxymethylcellulose, an aliginate, gelating, or polyvinyl pyrrolidone, a solution retardant such as paraffin, a resorption accelerator such as a quaternary salt and/or and absorption agent such as betonite, kaolin, or dicalcium phosphate. The powder mixture can be granulated by wetting with a binder such as syrup, starch paste, acadia mucilage, or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc, or mineral oil. The lubricated mixture is then compressed into tablets. The compounds of the present disclosure can also be combined with a free flowing inert carrier and compressed into tablets directly without going through the granulating or slugging steps. A clear or opaque protective coating consisting of a sealing coat of shellac, a coating of sugar or polymeric material, and a polish coating of wax can be provided. Dyestuffs can be added to these coatings to distinguish different unit dosages.

Oral fluids such as solution, syrups, and elixirs can be prepared in dosage unit form so that a given quantity contains a predetermined amount of the compound. Syrups can be prepared by dissolving the compound in a suitably flavored aqueous solution, while elixirs are prepared through the use of a non-toxic vehicle. Solubilizers and emulsifiers such as ethoxylated isostearyl alcohols and polyoxyethylene sorbitol ethers, preservatives, flavor additive such as peppermint oil or natural sweeteners, or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax, or the like.

The compounds of the invention, and pharmaceutically acceptable salts thereof, can also be administered in the form of liposome delivery systems, such as small unilamellar vesicles, large unilamellar vesicles, and multilamellar vesicles. Liposomes can be formed from a variety of phopholipids, such as cholesterol, stearylamine, or phophatidylcholines.

The compounds of the invention and pharmaceutically acceptable salts thereof may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethyleneoxidepolylysine substituted with palitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates, and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research 1986, 3(6), 318.

Pharmaceutical formulations adapted for topical administration may be formulated as ointments, creams, suspensions, lotions, powders, solutions, pastes, gels, sprays, aerosols, or oils.

Pharmaceutical formulations adapted for rectal administration may be presented as suppositories or as enemas.

Pharmaceutical formulations adapted for nasal administration wherein the carrier is a solid include a course powder having a particle size for example in the range 20 to 500 microns which is administered in the manner in which snuff is taken, i.e., by rapid inhalation through the nasal passage from a container of the powder held close up to the nose. Suitable formulations wherein the carrier is a liquid, for administration as a nasal spray or nasal drops, include aqueous or oil solutions of the active ingredient.

Pharmaceutical formulations adapted for administration by inhalation include fine particle dusts or mists, which may be generated by means of various types of metered, dose pressurized aerosols, nebulizers, or insufflators.

Pharmaceutical formulations adapted for vaginal administration may be presented as pessaries, tampons, creams, gels, pastes, foams, or spray formulations.

Pharmaceutical formulations adapted for parenteral administration include aqueous and non-aqueous sterile injection solutions which may contain anti-oxidants, buffers, bacteriostats, and soutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules, and tablets.

It should be understood that in addition to the ingredients particularly mentioned above, the formulations may include other agents conventional in the art having regard to the type of formulation in question, for example those suitable for oral administration may include flavoring agents.

The term "patient" includes both human and other mammals.

The term "treating" refers to: (i) preventing a disease, disorder or condition from occurring in a patient that may be predisposed to the disease, disorder, and/or condition but has not yet been diagnosed as having it; (ii) inhibiting the disease, disorder, or condition, i.e., arresting its development; and (iii) relieving the disease, disorder, or condition, i.e., causing regression of the disease, disorder, and/or condition.

The compounds of the present disclosure can also be administered with a cyclosporin, for example, cyclosporin A. Cyclosporin A has been shown to be active against HCV in clinical trials (Hepatology 2003, 38, 1282; Biochem. Biophys. Res. Commun. 2004, 313, 42; J. Gastroenterol. 2003, 38, 567).

Table 1 below lists some illustrative examples of compounds that can be administered with the compounds of this disclosure. The compounds of the disclosure can be administered with other anti-HCV activity compounds in combination therapy, either jointly or separately, or by combining the compounds into a composition.

**Table 1**

| *Brand Name* | *Physiological Class* | *Type of Inhibitor or Target* | *Source Company* |
|---|---|---|---|
| NIM811 | | Cyclophilin Inhibitor | Novartis |
| Zadaxin | | Immunomodulator | Sciclone |
| Suvus | | Methylene blue | Bioenvision |
| Actilon (CPG10101) | | TLR9 agonist | Coley |
| Batabulin (T67) | Anticancer | β-tubulin inhibitor | Tularik Inc., South San Francisco, CA |
| ISIS 14803 | Antiviral | antisense | ISIS Pharmaceutica ls Inc, Carlsbad, CA/Elan Pharmaceutical s Inc., New York, NY |
| Summetrel | Antiviral | antiviral | Endo Pharmaceutica ls Holdings Inc., Chadds Ford, PA |
| GS-9132 (ACH-806) | Antiviral | HCV Inhibitor | Achillion / Gilead |
| Pyrazolopyrimidine compounds and salts From WO-2005047288 26 May 2005 | Antiviral | HCV Inhibitors | Arrow Therapeutics Ltd. |
| Levovirin | Antiviral | IMPDH inhibitor | Ribapharm Inc., Costa Mesa, CA |
| Merimepodib (VX-497) | Antiviral | IMPDH inhibitor | Vertex Pharmaceutica ls Inc., Cambridge, MA |
| XTL-6865 (XTL-002) | Antiviral | monoclonal antibody | XTL Biopharmaceu ticals Ltd., Rehovot, Isreal |
| Telaprevir (VX-950, LY-570310) | Antiviral | NS3 serine protease inhibitor | Vertex Pharmaceutica ls Inc., Cambridge, MA/ Eli Lilly and Co. Inc., Indianapolis, IN |
| HCV-796 | Antiviral | NS5B Replicase Inhibitor | Wyeth / Viropharma |
| NM-283 | Antiviral | NS5B Replicase Inhibitor | Idenix / Novartis |
| GL-59728 | Antiviral | NS5B Replicase Inhibitor | Gene Labs / Novartis |
| GL-60667 | Antiviral | NS5B Replicase Inhibitor | Gene Labs / Novartis |
| 2'C MeA | Antiviral | NS5B Replicase Inhibitor | Gilead |
| PSI 6130 | Antiviral | NS5B Replicase Inhibitor | Roche |
| R1626 | Antiviral | NS5B Replicase Inhibitor | Roche |
| 2'C Methyl adenosine | Antiviral | NS5B Replicase Inhibitor | Merck |
| JTK-003 | Antiviral | RdRp inhibitor | Japan Tobacco Inc., Tokyo, Japan |
| Levovirin | Antiviral | ribavirin | ICN -Pharmaceutica ls, Costa Mesa, CA |
| Ribavirin | Antiviral | ribavirin | Schering-Plough Corporation, Kenilworth, NJ |
| Viramidine | Antiviral | Ribavirin Prodrug | Ribapharm Inc., Costa Mesa, CA |
| Heptazyme | Antiviral | ribozyme | Ribozyme Pharmaceutica ls Inc., Boulder, CO |
| BILN-2061 | Antiviral | serine protease inhibitor | Boehringer Ingelheim Pharma KG, Ingelheim, Germany |
| SCH 503034 | Antiviral | serine protease inhibitor | Schering Plough |
| Zadazim | Immune modulator | Immune modulator | SciClone Pharmaceutica ls Inc., San Mateo, CA |
| Ceplene | Immunomodulator | immune modulator | Maxim Pharmaceutica ls Inc., San Diego, CA |
| CellCept | Immunosuppressa nt | HCV IgG immunosuppressant | F. Hoffmann-La Roche LTD, Basel, Switzerland |
| Civacir | Immunosuppressa nt | HCV IgG immunosuppressant | Nabi Biopharmaceu ticals Inc., Boca Raton, FL |
| Albuferon - α | Interferon | albumin IFN-α2b | Human Genome Sciences Inc., Rockville, MD |
| Infergen A | Interferon | IFN alfacon-1 | InterMune Pharmaceutica ls Inc., Brisbane, CA |
| Omega IFN | Interferon | IFN-ω | Intarcia Therapeutics |
| IFN-β and EMZ701 | Interferon | IFN-β and EMZ701 | Transition Therapeutics Inc., Ontario, Canada |
| Rebif | Interferon | IFN-β1a | Serono, Geneva, Switzerland |
| Roferon A | Interferon | IFN-α2a | F. Hoffmann-La Roche LTD, Basel, Switzerland |
| Intron A | Interferon | IFN-α2b | Schering-Plough Corporation, Kenilworth, NJ |
| Intron A and Zadaxin | Interferon | IFN-α2b/α1-thymosin | RegeneRx B iopharmiceu ticals Inc., Bethesda, MD/ SciClone Pharmaceutica ls Inc, San Mateo, CA |
| Rebetron | Interferon | IFN-α2b/ribavirin | Schering-Plough Corporation, Kenilworth, NJ |
| Actimmune | Interferon | INF-γ | InterMune Inc., Brisbane, CA |
| Interferon-β | Interferon | Interferon-β-1a | Serono |
| Multiferon | Interferon | Long lasting IFN | Viragen/Valen tis |
| Wellferon | Interferon | lymphoblastoid IFN-αn1 | GlaxoSmithKl ine plc, Uxbridge, UK |
| Omniferon | Interferon | natural IFN-α | Viragen Inc., Plantation, FL |
| Pegasys | Interferon | PEGylated IFN-α2a | F. Hoffmann-La Roche LTD, Basel, Switzerland |
| Pegasys and Ceplene | Interferon | PEGytated IFN-α2a/ immune modulator | Maxim Pharmaceutica ls Inc., San Diego, CA |
| Pegasys and Ribavirin | Interferon | PEGylated IFN-α2a/ribavirin | F. Hoffmann-La Roche LTD, Basel, Switzerland |
| PEG-Intron | Interferon | PEGylated IFN-α2b | Schering-Plough Corporation, Kenilworth, NJ |
| PEG-Intron / Ribavirin | Interferon | PEGylated IFN-α2b/ribavirin | Schering-Plough Corporation, Kenilworth, NJ |
| IP-501 | Liver protection | antifibrotic | Indevus Pharmaceutica ls Inc., Lexington, MA |
| IDN-6556 | Liver protection | caspase inhibitor | Idun Pharmaceutica ls Inc., San Diego, CA |
| ITMN-191 (R-7227) | Antiviral | serine protease inhibitor | InterMune Pharmaceutica ls Inc., Brisbane, CA |
| GL-59728 | Antiviral | NS5B Replicase Inhibitor | Genelabs |
| ANA-971 | Antiviral | TLR-7 agonist | Anadys |

The compounds of the present disclosure may also be used as laboratory reagents. Compounds may be instrumental in providing research tools for designing of viral replication assays, validation of animal assay systems and structural biology studies to further enhance knowledge of the HCV disease mechanisms. Further, the compounds of the present disclosure are useful in establishing or determining the binding site of other antiviral compounds, for example, by competitive inhibition.

The compounds of this disclosure may also be used to treat or prevent viral contamination of materials and therefore reduce the risk of viral infection of laboratory or medical personnel or patients who come in contact with such materials, e.g., blood, tissue, surgical instruments and garments, laboratory instruments and garments, and blood collection or transfusion apparatuses and materials.

This disclosure is intended to encompass compounds of the invention when prepared by synthetic processes or by metabolic processes including those occurring in the human or animal body (*in vivo*) or processes occurring *in vitro*.

The abbreviations used in the present application, including particularly in the illustrative schemes and examples which follow, are well-known to those skilled in the art. Some of the abbreviations used are as follows: HATU for O-(7-azabenzotriazol-1-yl)-N,N,N',N'-tetramethyluronium hexafluorophosphate; Boc or BOC for tert-butoxycarbonyl; NBS for N-bromosuccinimide; tBu or t-Bu for tert-butyl; SEM for-(trimethylsilyl)ethoxymethyl; DMSO for dimethylsulfoxide; MeOH for methanol; TFA for trifluoroacetic acid; RT for room temperature or retention time (context will dictate); t_{R} for retention time; EDCI for 1-(3-dimethylaminopropyl)-3-ethylcarbodiimide hydrochloride; DMAP for 4-dimethylaminopyridine; THF for tetrahydrofuran; DBU for 1,8-diazabicyclo[5.4.0]undec-7-ene; t-Bu; DEA for diethylamine; HMDS for hexamethyldisilazide; DMF for N,N-dimethylformamide; Bzl for benzyl; EtOH for ethanol; iPrOH or i-PrOH for isopropanol; Me₂S for dimethylsulfide; Et₃N or TEA for triethylamine; Ph for phenyl; OAc for acetate; EtOAc for ethyl acetate; dppf for 1,1'-bis(diphenylphosphino)ferrocene; iPr₂EtN or DIPEA for diisopropylethylamine; Cbz for carbobenzyloxy; n-BuLi for n-butyllithium; ACN for acetonitrile; h or hr for hours; m or min for minutes; s for seconds; LiHMDS for lithium hexamethyldisilazide; DIBAL for diisobutyl aluminum hydride; TBDMSCl for tert-butyldimethylsilyl chloride; Me for methyl; ca. for about; OAc for acetate; iPr for isopropyl; Et for ethyl; Bn for benzyl; and HOAT for 1-hydroxy-7-azabenzotriazole.

The compounds and processes of the present disclosure will be better understood in connection with the following examples. Starting materials can be obtained from commercial sources or prepared by well-established literature methods known to those of ordinary skill in the art. It will be readily apparent to one of ordinary skill in the art that the compounds defined above can be synthesized by substitution of the appropriate reactants and agents in the syntheses shown below. It will also be readily apparent to one skilled in the art that the selective protection and deprotection steps, as well as the order of the steps themselves, can be carried out in varying order, depending on the nature of the variables to successfully complete the syntheses below.

### Compound analysis conditions

Purity assessment and low resolution mass analysis were conducted on a Shimadzu LC system coupled with Waters Micromass ZQ MS system. It should be noted that retention times may vary slightly between machines. The LC conditions employed in determining the retention time (RT) were:

| *Condition 1* | |
|---|---|
| Column | = Phenomenex-Luna 3.0X 50 mm S10 |
| Start %B | = 0 |
| Final %B | =100 |
| Gradient time | = 2 min |
| Stop time | = 3 min |
| Flow Rate | = 4 mL/min |
| Wavelength | = 220 nm |
| Slovent A | = 0.1 % TFA in 10% methanol/90%H₂O |
| Solvent B | = 0.1% TFA in 90% methanol/10% H₂O |
| | |

| *Condition 2* | |
|---|---|
| Column | = Phenomenex-Luna 4.6X50 mm S 10 |
| Start %B | = 0 |
| Final %B | = 100 |
| Gradient time | = 2 min |
| Stop time | = 3 min |
| Flow Rate | = 5 mL/min |
| Wavelength | = 220 nm |
| Slovent A | = 0.1 % TFA in 10% methanol/90%H₂O |
| Solvent B | = 0.1% TFA in 90% methanol/10% H₂O |
| | |

| *Condition 3* | |
|---|---|
| Column | = HPLC XTERRA C18 3.0 x 50mm S7 |
| Start %B | = 0 |
| Final %B | = 100 |
| Gradient time = | 3 min |
| Stop time | = 4 min |
| Flow Rate | = 4 mL/min |
| Wavelength | = 220 nm |
| Slovent A | = 0.1% TFA in 10% methanol/90%H₂O |
| Solvent B | = 0.1% TFA in 90% methanol/10% H₂O |
| | |

| *Condition M1* | |
|---|---|
| Column: Luna 4.6X 50 mm S10 | |
| Start %B | = 0 |
| Final %B | = 100 |
| Gradient time | = 3 min |
| Stop time | = 4 min |
| Flow rate | = 4 mL/min |
| Solvent A: | = 95% H₂0: 5% CH₃CN, 10 mm Ammonium acetate |
| Solvent B: | = 5% H₂O : 95% CH₃CN; 10 mm Ammonium acetate |

### Reference Example 1, Step b

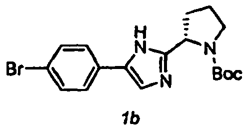

A mixture of ketoamide 1a (12.8 g, 31.12 mmol) and NH₄OAc (12.0 g, 155.7 mmol) in xylenes (155 mL) was heated in a sealed tube at 140°C for 2 hours. The volatile component was removed *in vacuo*, and the residue was partitioned carefully between ethyl acetate and water, whereby enough saturated NaHCO₃ solution was added so as to make the pH of the aqueous phase slightly basic after the shaking of the biphasic system. The layers were separated, and the aqueous layer was extracted with an additional ethyl acetate. The combined organic phase was washed with brine, dried (MgSO₄), filtered, and concentrated *in vacuo*. The resulting material was recrystallized from ethyl acetate/hexanes to provide two crops of imidazole 1b as a light-yellow dense solid, weighing 5.85 g. The mother liquor was concentrated *in vacuo* and submitted to a flash chromatography (silica gel; 30% ethyl acetate/hexanes) to provide an additional 2.23 g of imidazole 1b. ¹H NMR (DMSO-d₆, δ = 2.5 ppm, 400 MHz): δ 12.17/11.92/11.86 (m, 1H), 7.72-7.46/7.28 (m, 5H), 4.86-4.70 (m, 1H), 3.52 (app br s, 1H), 3.36 (m, 1H), 2.30-1.75 (m, 4H), 1.40/1.15 (app br s, 9H). LC (Cond. 1): RT =1.71 min; >98% homogeneity index; LC/MS: Anal. Calcd. for [M+H]⁺ C₁₈H₂₃BrN₃O₂:392.10; found 391.96; HRMS: Anal. Calcd. for [M+H]⁺ C₁₈H₂₃BrN₃O₂: 392.0974; found 392.0959

The optical purity of the two samples of 1b were assessed using the chiral HPLC conditions noted below (ee > 99% for the combined crops; ee = 96.7% for the sample from flash chromatography):
Column: Chiralpak AD, 10 um, 4.6 x 50 mm
Solvent: 2% ethanol/heptane (isocratic)
Flow rate: 1 mL/min
Wavelength: either 220 or 254 nm
Relative retention time: 2.83 minutes (R), 5.34 minutes (S)
Analogous compounds can be prepared by incorporating the appropriate ketoamide.

### Reference Example 1, Step c

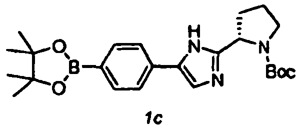

Pd(Ph₃P)₄ (469 mg, 0.406 mmol) was added to a pressure tube containing a mixture of bromide 1b (4.008 g, 10.22 mmol), bis(pinacolato)diboron (5.422 g, 21.35 mmol), potassium acetate (2.573g, 26.21 mmol) and 1,4-dioxane (80 mL). The reaction flask was purged with nitrogen, capped and heated with an oil bath at 80°C for 16.5 hours. The reaction mixture was filtered and the filtrate was concentrated *in vacuo*. The crude material was partitioned carefully between CH₂Cl₂ (150 mL) and an aqueous medium (50 mL water + 10 mL saturated NaHCO₃ solution). The aqueous layer was extracted with CH₂Cl₂, and the combined organic phase was dried (MgSO₄), filtered, and concentrated *in vacuo*. The resulting material was purified with flash chromatography (sample was loaded with eluting solvent; 20-35% ethyl acetate/CH₂Cl₂) to provide boronate 1c, contaminated with pinacol, as an off-white dense solid; the relative mole ratio of 1c to pinacol was about 10:1 (¹H NMR). The sample weighed 3.925 g after ~2.5 days exposure to high vacuum. ¹H NMR (DMSO-d₆ δ= 2.5 ppm, 400 MHz): 12.22/11.94/11.87 (m, 1H), 7.79-7.50/7.34-7.27 (m, 5H), 4.86-4.70 (m, 1H), 3.52 (app br s, 1H), 3.36 (m, 1H), 2.27-1.77 (m, 4H), 1.45-1.10 (m, 21H). LC (Cond. 1): RT = 1.64 min; LC/MS: Anal. Calcd. for [M+H]⁺ C₂₄H₃₅BN₃O₄· 440.27; found 440.23.
Analogous compounds can be prepared by incorporating the appropriate aryl bromide.

### Reference Example 2, Step a-b

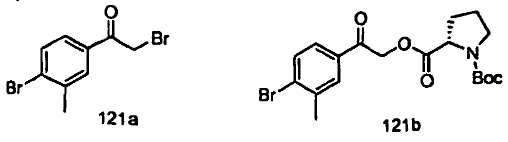

PdCl₂(Ph₃P)₂ (257 mg, 0.367 mmol) was added to a dioxane (45 mL) solution of 1-bromo-4-iodo-2-methylbenzene (3.01 g, 10.13 mmol) and tri-n-butyl(1-ethoxyvinyl)stannane (3.826 g, 10.59 mmol) and heated at 80°C for ~17 hours. The reaction mixture was treated with water (15 mL), cooled to -0 °C (ice/water), and then NBS (1.839 g, 10.3 mmol) was added in batches over 7 minutes. After about 25 minutes of stirring, the volatile component was removed *in vacuo*, and the residue was partitioned between CH₂Cl₂ and water. The aqueous layer was extracted with CH₂Cl₂, and the combined organic phase was dried (MgSO₄), filtered, and concentrated *in vacuo*. The resulting crude material was purified by a gravity chromatography (silica gel; 4% ethyl acetate/hexanes)to provide bromide 121a as a brownish-yellow solid (2.699 g); the sample is impure and contains stannane-derived impurities, among others. ¹H NMR (CDCl₃, δ = 7.24, 400 MHz): 7.83 (s, 1H), 7.63 (s, 2H), 4.30 (s, 2H), 2.46 (s, 3H).

A CH₃CN (15 mL) solution of 121a (2.69 g, < 9.21 mmol) was added dropwise over 3 minutes to a CH₃CN (30 mL) solution of (*S*)-Boc-proline (2.215 g, 10.3 mmol) and triethylamine (1.40 mL, 10.04 mmol), and stirred for 90 minutes. The volatile component was removed *in vacuo*, and the residue was partitioned between water and CH₂Cl₂, and the organic phase was dried (MgSO₄), filtered, and concentrated *in vacuo*. The resulting crude material was purified by a flash chromatography (silica gel; 15-20% ethyl acetate/hexanes) to provide 121b as a colorless viscous oil (2.74g). ¹H NMR (DMSO-d₆, δ = 2.50, 400 MHz): δ 7.98 (m, 1H), 7.78 (d, *J* = 8.3, 1H), 7.72-7.69 (m, 1H), 5.61-5.41 (m, 2H), 4.35-4.30 (m, 1H), 3.41-3.30 (m, 2H), 2.43 (s, 3H), 2.33-2.08 (m, 2H), 1.93-1.83 (m, 2H), 1.40/1.36 (s, 9H); LC (Cond. 1): RT = 1.91 min; >95% homogeneity index; LC/MS: Anal. Calcd. for [M+Na]⁺ C₁₉H₂₄BrNNaO₅ 448.07; found 448.10.
Additional keto-esters can be prepared in analogous fashion.
LC conditions: *Condition 1*: Phenomenex LUNA C-18 4.6 x 50 mm, 0 to 100% B over 3 minutes, 1 minute hold time, A = 90% water, 10% methanol, 0.1% TFA, B = 10% water, 90% methanol, 0.1% TFA, 220nm, 5 µL injection volume.
*Condition 2*: Phenomenex LUNA C-18 4.6 x 50 mm, 0 to 100% B over 2 minutes, I minute hold time, A = 90% water, 10% methanol, 0.1% TFA, B =10% water, 90% methanol, 0.1% TFA, 220nm, 5 µL injection volume.

### Section R

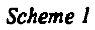

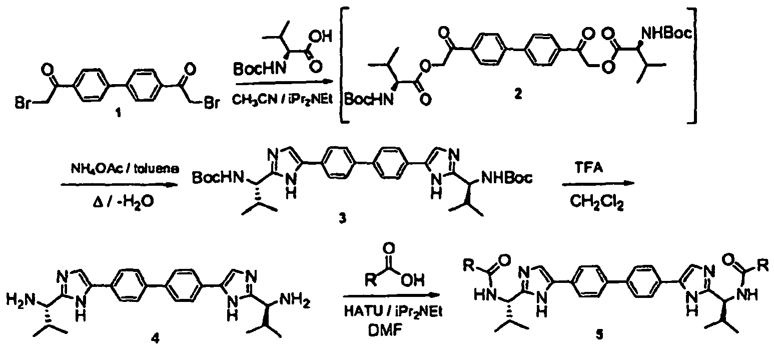

### Example R1. tert-butyl (1S,1'S)-1,1'-(5,5'-(biphenyl-4,4'-diyl)bis(1H-imidazole-5,2-diyl))bis(2-methylpropane,1,1-diyl)dicarbamate (3).

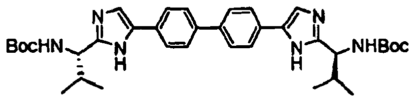

A solution of 1,1'-(biphenyl-4,4'-diyl)bis(2-bromoethanone) (1) (5.14 g, 18.4 mmol) and N-Boc-L-Valine (8.00 g, 36.8 mmol) in dry CH₃CN (40 mL) was treated with iPr₂NEt (7.05 mL, 40.5 mmol) and the solution was allowed to stir at rt overnight. The solvent was removed in vacuo and the resulting slurry was partitioned between EtOAc and H₂O. The layers were separated and the aq phase was extracted with EtOAc. The combined organic layers were dried over MgSO₄, filtered, and concentrated in vacuo. The residue was suspended in toluene (100 mL) and NH₄OAc (14.2 g, 184.2 mmol) was added. The mixture was heated reflux for 16 h with azeotropic removal of H₂O (Dean-Stark). The mixture was cooled to rt and concentrated to dryness in vacuo. The residue was purified column chromatography (biotage) eluting with 0 to 40% CH₃CN in CH₂Cl₂ and then 10% MeOH in CH₂Cl₂ to afford the title compound (5.77 g, 50%) as a yellow foam. ¹HNMR (500 MHz, DMSO-d₆) δ 11.77 - 11.93 (m, 2H), 7.80 (s, br, 4H), 7.66 - 7.68 (m, 4H), 7.53 (s, br, 2H), 6.89 (d, J = 7.7 Hz, 2H), 4.43 (app t, J = 8.2 Hz, 2H), 2.05 - 2.11 (m, 2H), 1.38 (s, 18H), 0.90 (d, J = 6.6 Hz, 6H), 0.78 (d, J = 6.6 Hz, 6H). LCMS: Anal. Calcd. for C₃₆H₄₈N₆O₄: 628; found: 629 (M+H)⁺.

### Example R2. tert-butyl (1R,1'R)-1,1'-(5,5'-(biphenyl-4,4'-diyl)bis(1H-imidazole-5,2-diyl))bis(2-methylpropane-1,1-diyl)dicarbamate.

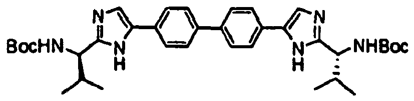

The title compound was prepared according to the method in Example R1 starting with 1,1'-(biphenyl-4,4'-diyl)bis(2-bromoethanone) (1) and N-Boc-D-Valine. LCMS: Anal. Calcd. for C₃₆H₄₈N₆O₄: 628; found: 629 (M+H)⁺.

### Example R3. (1S,1'S)-1,1'-(5,5'-(biphenyl-4,4'-diyl)bis(1H-imidazole-5,2-diyl))bis(2-methylpropan-1-amine).

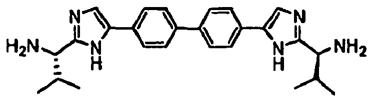

A solution of tert-butyl (1R,1'R)-1,1'-(5,5'-(biphenyl-4,4'-diyl)bis(1H-imidazole-5,2-diyl))bis(2-methylpropane-1,1-diyl)dicarbamate (1.00 g, 1.59 mmol) in CH₂Cl₂ (30 mL) was added TFA (20 mL) and the solution was allowed to stir at rt for 5 h. The solvents were removed in vacuo, the residue was dissolved in CH₂Cl₂: MeOH (1:1) (ca. 10 mL) and absorbed onto an MCX cation exchange cartridge (Oasis). The resin was washed with MeOH and the desired material was released by elution with NH₃ in MeOH (2M). The solvents were removed in vacuo to afford the title compound (0.622 g, 91 %) as a yellow foam which was sufficiently pure for use in subsequent steps. ¹HNMR (300 MHz, CD₃OD) δ 7.76 (app d, J = 8.4 Hz, 4H), 7.68 (app d, J = 8.4 Hz, 4H), 7.38 (s, 2H), 3.77 (d, J = 7.3 Hz, 2H), 2.07 (sept, J = 7.0 Hz, 2H), 1.03 (d, J = 7.0 Hz, 6H), 0.88 (d, J = 7.0 Hz, 6H). LCMS: Anal. Calcd. for C₂₆H₃₂N₆: 428; found: 429 (M+H)⁺.

### Example R4. (1R,1'R)-1,1'-(5,5'-(biphenyl-4,4'-diyl)bis(1H-imidazole-5,2-diyl))bis(2-methylpropan-1-amine).

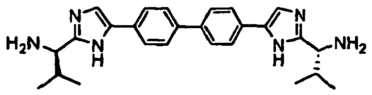

The title compound was prepared according to the method given in Example R3 starting with tert-butyl (1R,1'R)-1,1'-(5,5'-(biphenyl-4,4'-diyl)bis(1H-imidazole-5,2-diyl))bis(2-methylpropane-1,1-diyl)dicarbamate. LCMS: Anal. Calcd. for C₂₆H₃₂N₆:428; found: 429 (M+H)⁺.

### Example R5: dimethyl (4,4'-biphenyldiylbis(1H-imidazole-4,2-diyl((1S)-2-methyl-1,1-propanediyl)imino((1S)-1-oxo-1,2-propanediyl)))biscarbamate

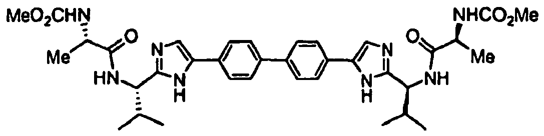

A solution of (1S,1'S)-1,1'-(5,5'-(biphenyl-4,4'-diyl)bis(1H-imidazole-5,2-diyl))bis(2-methylpropan-1-amine) (120 mg, 0.280 mmol), (S)-2-(methoxycarbonylamino)propanoic acid (124 mg, 0.840 mmol) and iPr₂NEt (0.489 mL, 2.80 mmol) in DMF (5 mL) was treated with HATU (426 mg, 1.12 mmol) and the reaction mixture was stirred at rt for 2h. The reaction mixture was partitioned between H₂O and EtOAc, the layers separated and the aq phase was extracted with EtOAc. The combined organic layers were concentrated in vacuo and purified by column chromatography (Biotage, 25S eluting with 0 to 40% CH₃CN in CH₂Cl₂ and then 10% MeOH in CH₂Cl₂. The appropriate fractions were concentrated in vacuo and the residue was purified by prep HPLC (CH₃CN-H₂O-TFA). Two subsequent purifications by prep HPLC (CH₃CN-H₂O-NH₄OAc) followed by a final prep HPLC (CH₃CN-H₂O-TFA) afforded the TFA salt of the title compound (4.9 mg, 2%) as a colorless solid. ¹HNMR (300 MHz, CD₃OD) δ 7.89 (s, 2H), 7.86 (s, 8H), 4.87 (d, J = 8.5 Hz, 2H), 4.19 (q, J = 7.0 H₂, 2H), 3.65 (s, 6H), 1.33 (d, J = 7.0 Hz, 6H), 1.14 (d, J = 7.0 Hz, 6H), 0.96 (d, J = 7.0 Hz, 6H). LCMS: Anal. Calcd. for C₃₆H₄₆N₈O₆: 686; found: 687 (M+H)⁺.

**Table 1A. The following were prepared similary starting with the appropriate amine and substitute amino acid.**

| *Example* | *Compound Name* | *Structure* | *Analytical Data* |
|---|---|---|---|
| Example R6 | dimethyl (4,4'-biphenyldiylbis(1H-imidazole-4,2-diyl((1S)-2-methyl-1,1-propanediyl)imino(( 2S,3R)-3-methoxy-1-oxo-1,2-butanediyl)))biscarb amate | 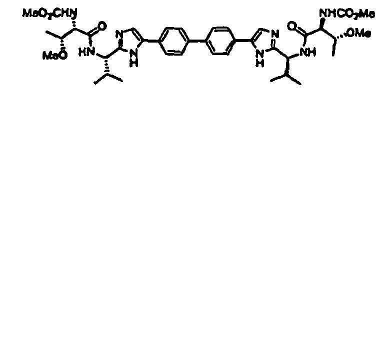 | LCMS: Anal. Calcd. for C₄₀H₅₄N₈O₈: 774; found: 775 (M+H)⁺. |
| Example R7 | dimethyl (4,4'-biphenyldiylbis(1H-imidazole-4,2-diyl((1S)-2-methyl-1,1-propanediyl)imino(( 2S)-3-methyl-1-oxo-1,2-butanediyl)))biscarb amate | 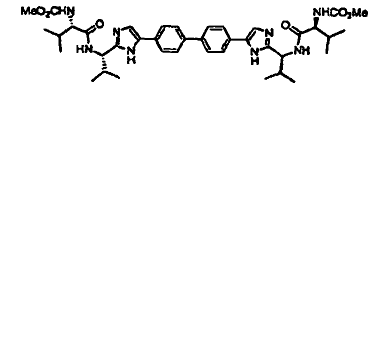 | LCMS: Anal. Calcd. for C₄₄H₅₄N₈O₆: 742; found: 743 (M+H)⁺. |
| Example R8 | dimethyl (4,4'-biphenyldiylbis(1H-imidazole-4,2-diyl((1S)-2-methyl-1,1-propanediyl)imino(( 2S)-4-methoxy-1-oxo-1,2-butanediyl)))biscarb amate | 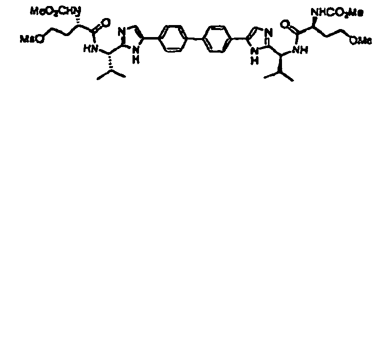 | LCMS: Anal. Calcd. for C₄₀H₅₄N₈O₈: 774; found: 775 (M+H)⁺. |
| Example R9 | dimethyl (4,4'-biphenyldiylbis(1H-imidazole-4,2-diyl((1R)-2-methyl-1,1-propanediyl)imino(( 2S)-3-methyl-1-oxo-1,2-butanediyl)))biscarb amate | 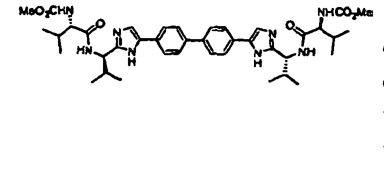 | LCMS: Anal. Calcd. for C₄₀H₅₄N₈O₆: 742; found: 743 (M+H)⁺. |
| Example R10 | dimethyl (4,4'-biphenyldiylbis(1H-imidazole-4,2-diyl((1R)-2-methyl-1,1-propanediyl)imino(( 2S)-1-oxo-1,2-propanediyl)))biscar bamate | 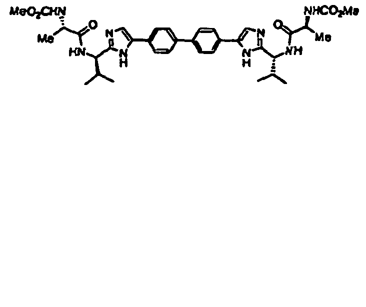 | LCMS: Anal. Calcd. for C₃₆H₄₆N₈O₆ 686; found: 687 (M+H)⁺. |
| Example R11 | dimethyl (4,4'-biphenyldiylbis(1H-imidazole-4,2-diyl((1R)-2-methyl-1,1-propanediyl)imino(( 2S)-4-methoxy-1-oxo-1,2-butanediyl)))biscarb amate | 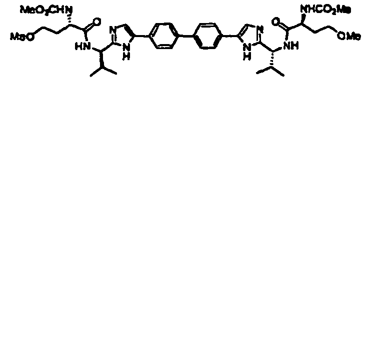 | LCMS: Anal. Calcd. for C₄₀H₅₄N₈O₈: 774; found: 775 (M+H)⁺. |
| Example R12 | dimethyl (4,4'-biphenyldiylbis(1H-imidazole-4,2-diyl((1R)-2-methyl-1,1-propanediyl)imino(( 2S,3R)-3-methoxy-1-oxo-1,2-butanediyl)))biscarb amate | 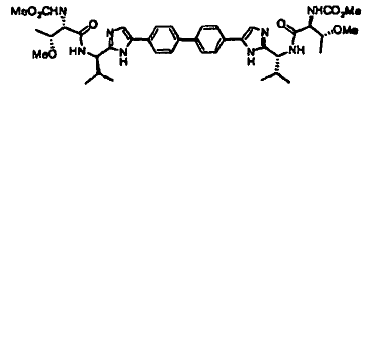 | LCMS: Anal. Calcd. for C₄₀H₅₄N₈O₈: 774; found: 775 (M+H)⁺. |
| Example R13 | dimethyl (4,4'-biphenyldiylbis(1H-imidazole-4,2-diyl((1R)-2-methyl-1,1-propinediyl)imino(( 2R)-3-methyl-1-oxo-1,2-butanediyl)))biscarb amate | 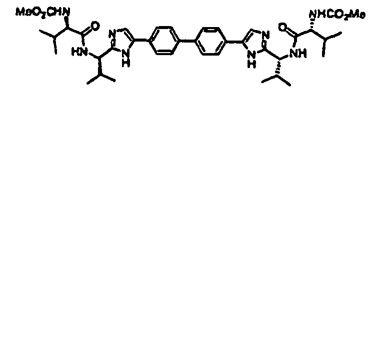 | Anal. Calcd. for C₄₀H₅₄N₈O₆: 742; found: 743 (M+H)⁺. |
| Example R14 | dimethyl (4,4'-biphenyldiylbis(1H-imidazole-4,2-diyl((1 S)-2-methyl-1,1-propanediyl)imino(( 2R)-3-methyl-1-oxo-1,2-butanediyl)))biscarb amate | 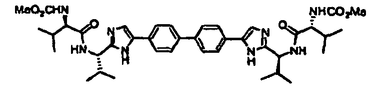 | Anal. Calcd. for C₄₀H₅₄N₈O₆: 742; found: 743 (M+H)⁺. |

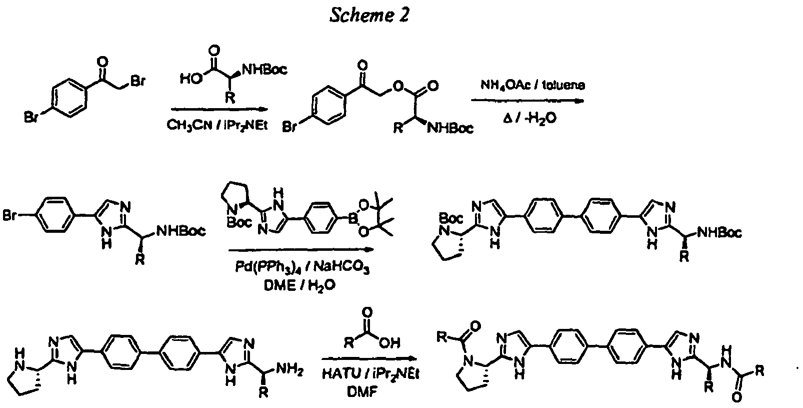

### Example R15. (S)-tert-butyl 1-(5-(4-bromophenyl)-1H-imidazol-2-yl)-2-methylpropylcarbamate.

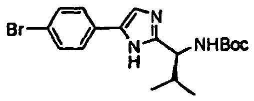

A solution of 2-bromo-1-(4-bromophenyl)ethanone (6.23 g, 22.3 mmol) and N-Boc-L-Valine (5.00 g, 23.0 mmol) in dry CH₃CN (30 mL) was treated with iPr₂NEt (4.40 mL, 25.3 mmol) and the solution was allowed to stir at rt overnight. The solvent was removed in vacuo and the resulting slurry was partitioned between EtOAc and H₂O. The layers were separated and the aq phase was extracted with EtOAc. The combined organic layers were dried over MgSO₄, filtered, and concentrated in vacuo to give a yellow oil. LCMS: Anal. Calcd. for C₁₈H₂₄BrNO₅: 413, 415; found: 412,414 (M-H)⁻. The residue was suspended in toluene (60 mL) and NH₄OAc (8.61 g, 111.7 mmol) was added. The mixture was heated reflux for 16 h with azeotropic removal of H₂O (Dean-Stark). The mixture was cooled to rt and concentrated to dryness in vacuo. The residue was purified column chromatography (biotage) eluting with 10% MeOH in CH₂Cl₂ to afford the title compound (8.55 g, 94%) as a yellow foam. ¹HNMR (500 MHz, CD₃OD) δ 7.84 (s, 1H), 7.66 (app d, J = 8.9 Hz, 2H), 7.64 (app d, J = 8.9 Hz, 2H), 4.65 (d, J = 7.7 Hz, 1H), 2.23 - 2.27 (m, 1H), 1.42 (s, 9H), 1.07 (d, J = 6.5 Hz, 3H), 0.93 (d, J= 6.5 Hz, 3H). LCMS: Anal. Calcd. for C₁₈H₂₄BrN₃O₂: 393, 395; found: 394, 396 (M+H)⁺.

### Example R16. (R)-tert-butyl 1-(5-(4-bromophenyl)-1H-imidazol-2-yl)-2-methylpropylcarbamate.

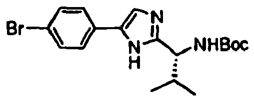

The title compound was prepared starting from 2-bromo-1-(4-bromophenyl)ethanone and N-Boc-D-Valine using the method given in Example R15. ¹HNMR (500 MHz, CD₃OD) δ 7.83 (s, 1H), 7.68 (app d, J = 8.7 Hz, 2H), 7.64 (app d, J = 8.7 Hz, 2H), 4.65 (d, J = 7.3 Hz, 1H), 2.22 -2.26 (m, 1H), 1.42 (s, 9H), 1.08 (d, J = 6.7 Hz, 3H), 0.93 (d, J = 6.7 Hz, 3H). LCMS: Anal. Calcd. for C₁₈H₂₄BrN₃O₂: 393, 395; found: 394, 396 (M+H)⁺.

### Example R17. (S)-tert-butyl 2-(5-(4'-(2-((S)-1-(tert-butoxycarbonylamino)-2-methylpropyl)-1H-imidazol-5-yl)biphenyl-4-yl)-1H-imidazol-2-yl)pyrrolidine-1-carboxylate.

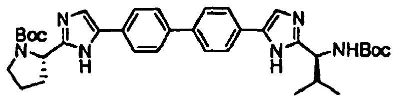

A solution of (S)-tert-butyl 1-(5-(4-bromophenyl)-1H-imidazol-2-yl)-2-methylpropylcarbamate (2.00 g, 5.07 mmol), (S)-tert-butyl 2-(5-(4-(4,4,5,5-tetramethyl-1,3,2-dioxaborolan-2-yl)phenyl)-1H-imidazol-2-yl)pyrrolidine-1-carboxylate (2.23 g, 5.07 mmol) and NaHCO₃ (1.49 g, 17.8 mmol) in DME (50 mL) and Water (15 mL) was degassed by evacuating and backfilling with N₂.

Tetrakis(triphenylphosphine)palladium(0) (0.293 g, 0.254 mmol) was added and the the reaction mixture was heated overnight at 100°C. The mixture was cooled to rt and the DME was removed in vacuo. The residue was partitioned between H₂O and EtOAc and the layers separated. The aq phase was extracted with EtOAc and the combined organic layers were concentrated to dryness in vacuo and purified purified by column chromatography (Biotage) eluting with a gradient of 0 to 40% CH₃CN in CH₂Cl₂ and then 0 to 10% MeOH in CH₂Cl₂. The title compound (2.05 g, 65 % yield) was obtained as a brown foam. This material was used as is in subsequent steps. LCMS: Anal. Calcd. for C₃₆H₄₆N₆O₄:626; found: 627 (M+H)⁺.

### Example R17A. (S)-2-methyl-1-(5-(4'-(2-((S)-pyrrolidin-2-yl)-1H-imidazol-5-yl)biphenyl-4-yl)-1H-imidazol-2-yl)propan-1-amine.

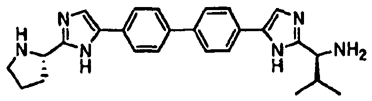

To a solution of (S)-tert-butyl 2-(5-(4'-(2-((S)-1-(tert-butoxycarbonylamino)-2-methylpropyl)-1H-imidazol-5-yl)biphenyl-4-yl)-1H-imidazol-2-yl)pyrrolidine-1-carboxylate (2.05 g, 3.27 mmol) in CH₂Cl₂ (20 mL) was added TFA (5 mL, 64.9 mmol) and the solution was allowed to stir for 2h. The solvents were removed in vacuo, the residue was dissolved in CH₂Cl₂ : MeOH (1:1) (ca. 10 mL) and absorbed onto an MCX cation exchange cartridge (Oasis). The resin was washed with MeOH and the desired material was released by elution with NH₃ in MeOH (2M). The solvents were removed in vacuo to afford the title compound (0.622 g, 91%) as a yellow foam which was sufficiently pure for use in subsequent steps. ¹HNMR (500 MHz, CD₃OD) δ 7.85 - 7.88 (m, 4H), 7.75 (m, app d, J = 8.2 Hz, 4H), 7.70 (app d, J = 5.8 Hz, 2H), 4.96 (t, J = 8.2 Hz, 1H), 4.30 (d, J = 8.5 Hz, 1H), 3.54 - 3.57 (m, 1H), 3.47 - 3.52 (m, 1H), 2.55 - 2.62 (m, 1H), 2.38 - 2.46 (m, 1H), 2.28 - 2.36 (m, 1H), 2.15 - 2.24 (m, 1H), 1.17 (d, J = 6.7 Hz, 3H), 0.94 (d, J = 6.7 Hz, 3H). LCMS: Anal. Calcd. for C₂₆H₃₀N₆: 426; found: 427 (M+H)⁺.

### Example R18. N²-(methoxycarbonyl)-N-((1S)-1-(4-(4'-(2-((2S)-1-(N-(methoxycarbonyl)-L-valyl)-2-pyrrolidinyl)-1H-imidazol-4-yl)-4-biphenylyl)-1H-imidazol-2-yl)-2-methylpropyl)-L-valinamide

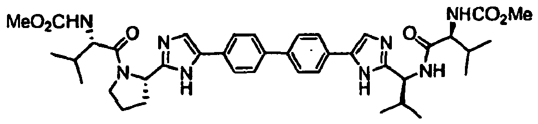

A single neck 50 mL flask equipped with a magnetic stirrer was charged with (S)-2-methyl-1-(5-(4'-(2-((S)-pyrrolidin-2-yl)-1H-imidazol-5-yl)biphenyl-4-yl)-1H-imidazol-2-yl)propan-1-amine (80 mg, 0.188 mmol) and (S)-2-(methoxycarbonylamino)-3-methylbutanoic acid (99 mg, 0.563 mmol) in DMF (5 mL) and DIEA (0.328 mL, 1.875 mmol) was added. To this solution was added HATU (285 mg, 0.750 mmol) added and the reaction was allowed to stir for 4h rt. The reaction mixture was partitioned between H₂O and EtOAc, the layers separated and the aq phase was extracted with EtOAc. The combined organic layers were concentrated in vacuo and purified by column chromatography (Biotage, 25S) eluting with 0 to 40% CH₃CN in CH₂Cl₂ and then 10% MeOH in CH₂Cl₂. The appropriate fractions were concentrated in vacuo and the residue was purified by prep HPLC (CH₃CN-H₂O-TFA) and then a subsequent purification by prep HPLC (CH₃CN-H₂O-NH₄OAc) gave the title compound (31.8 mg, 23%) as a colorless solid. ¹HNMR (500 MHz, CD₃OD) δ 7.71 (m, 4H), 7.64 - 7.67 (m, 4H), 7.35 (s, 1H), 7.32 (s, 1H), 5.33 - 5.34 (m, 0.2H), 5.17 (dd, J = 7.6, 5.5 Hz, 0.8H), 4.77 (d, J = 9.2 Hz, 1H), 4.22 and 4.10 (d, J = 7.6 Hz, 1H, rotamers 4:1 ratio), 3.97 - 4.01 (m, 1H), 3.94 (d, J = 7.0 Hz, 1H), 3.84 - 3.89 (m, 1H), 3.64 (s, 6H), 2.28 - 2.35 (m, 1H), 2.18 - 2.27 (m, 3H), 1.98 - 2.08 (m, 3H), 1.05 (d, J = 6.7 Hz, 3H), 0.98 and 0.94 (d, J = 6.7 Hz, 3H), 0.90 (d, J = 6.7 Hz, 6H), 0.88 (d, J = 6.7 Hz, 3H), 0.86 (d, J = 6.7 Hz, 3H). LCMS: Anal. Calcd. for C₄₀H₅₂N₈O₆: 740; found: 741 (M+H)⁺.

**Table 2A. The following were prepared similary starting with the appropriate amine and substitute amino acid.**

| *Example* | *Compound Name* | *Structure* | *Analytical Data* |
|---|---|---|---|
| Example R19 | methyl ((1S,2R)-2-methoxy-1-(((2S)-2-(4-(4'-(2-((1S)-1-((N-(methoxycarbonyl)-O-methyl-L-threonyl)amino)-2-methylpropyl)-1H-imidazol-4-yl)-4-biphenylyl)-1H-imidazol-2-yl)-1-pyrrolidinyl)carbonyl) propyl)carbamate | 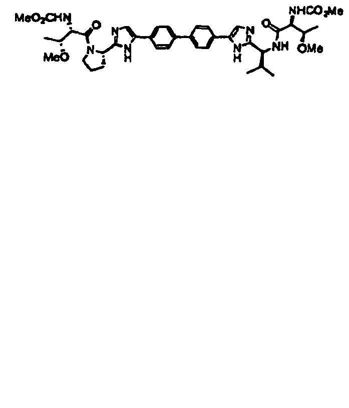 | LCMS: Anal. Calcd. for C₄₀H₅₂N₈O₈: 772 found: 773 (M+H)⁺. |
| Example R20 | methyl ((1S)-3-methoxy-1-(((2S)-2-(4-(4'-(2-((1S)-1-((N-(methoxycarbonyl)-O-methyl-L-homoseryl)amino)-2-methylpropyl)-1H-imidazol-4-yl)-4-biphenylyl)-1H-imidazol-2-yl)-1-pyrrolidinyl)carbonyl) propyl)carbamate | 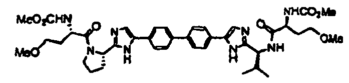 | LCMS: Anal. Calcd. for C₄₀H₅₂N₈O₈: 772 found: 773 (M+H)⁺. |
| Example R21 | methyl ((1S)-2-((2S)-2-(4-(4'-(2-((1S)-1-((N-(methoxycarbonyl)-L-alanyl)amino)-2-methylpropyl)-1H-imidazol-4-yl)-4-biphenylyl)-1H-imidazol-2-yl)-1-pyrrolidinyl)-1-methyl-2-oxoethyl)carbamate | 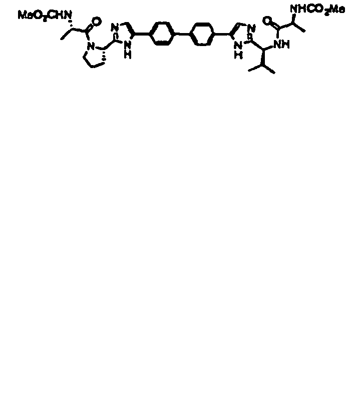 | LCMS: Anal. Calcd. for C₃₆H₄₄N₈O₆: 684; found: 685 (M+H)⁺. |
| Example R22 | N²-(methoxycarbonyl)-N-((1R)-1-(4-(4'-(2-((2S)-1-(N-(methoxycarbonyl)-L-valyl)-2-pyrrolidinyl)-1H-imidazol-4-yl)-4-biphenylyl)-1H-imidazol-2-yl)-2-methylpropyl)-L-valinamide | 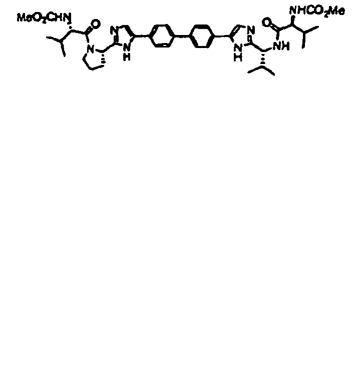 | LCMS: Anal. Calcd. for C₄₀H₅₂N₈O₆: 740; found: 741 (M+H)⁺. |
| Example R23 | methyl ((1S,2R)-2-methoxy-1-(((2S)-2-(4-(4'-(2-((1R)-1-((N-(methoxycarbonyl)-O-methyl-L-threonyl)amino)-2-methylpropyl)-1H-imidazol-4-yl)-4-biphenylyl)-1H-imidazol-2-yl)-1-pyrrolidinyl)carbonyl) propyl)carbamate | 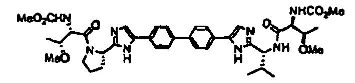 | LCMS: Anal. Calcd. for C₄₀H₅₂N₈O₈: 772 found: 773 (M+H)⁺. |
| Example R24 | methyl ((1S)-3-methoxy-1-(((2S)-2-(4-(4'-(2-((1R)-1-((N-(methoxycarbonyl)-O-methyl-L-homoseryl)amino)-2-methylpropyl)-1H-imidazol-4-yl)-4-biphenylyl)-1H-imidazol-2-yl)-1-pyrrolidinyl)carbonyl) propyl)carbamate | 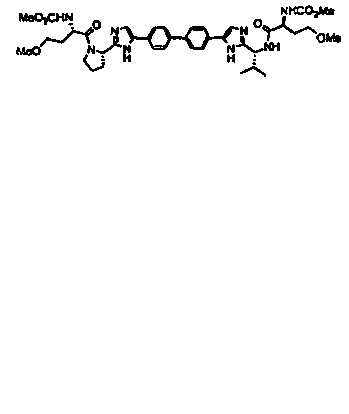 | LCMS: Anal. Calcd. for C₄₀H₅₂N₈O₈: 772; found: 773 (M+H)⁺. |
| Example R25 | methyl ((1S)-2-((2S)-2-(4-(4'-(2-((1R)-1-((N-(methoxycarbonyl)-L-alanyl)amino)-2-methylpropyl)-1H-imidazol-4-yl)-4-biphenylyl)-1H-imidazol-2-yl)-1-pyrrolidinyl)-1-methyl-2-oxoethyl)carbamate | 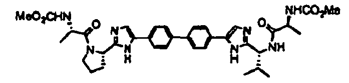 | LCMS: Anal. Calcd. for C₃₆H₄₄N₈O₆: 684; found: 685 (M+H)⁺. |

| | | | |
|---|---|---|---|
| 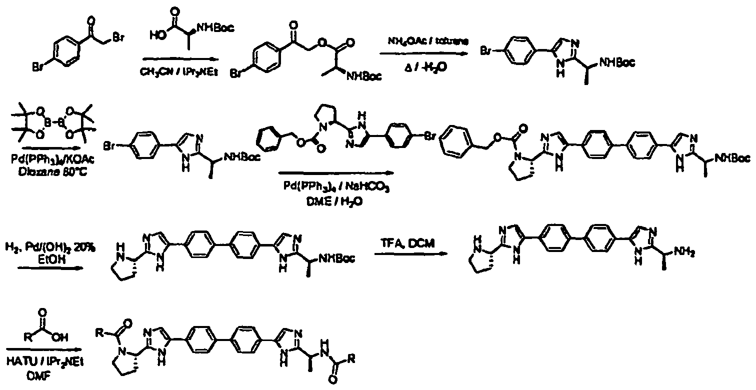 | | | |

| *Example* | *Structure* | | *Analytical Data* |
|---|---|---|---|
| Example A | 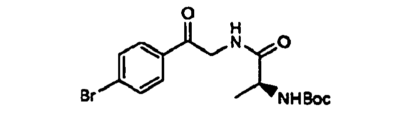 | | LCMS: Anal. Calcd. for C₁₆H₂₁BrN₂O₄:385 found: 386(M+H)⁺. |
| Example B | 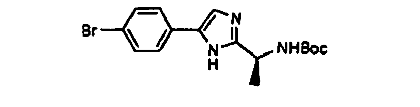 | | LCMS: Anal. Calcd. for C₁₆H₂₁BrN₃O₂: 366 found: 367 (M+H)⁺. |
| Example C | 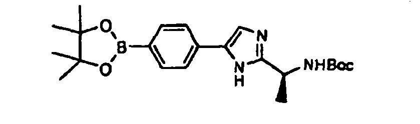 | | LCMS: Anal. Calcd. for C₂₂H₃₁BN₃O₄:413 ; found: 414 (M+H)⁺. |
| Example D | 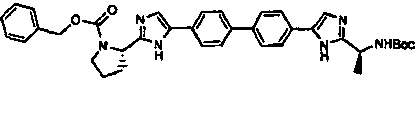 | | LCMS: Anal. Calcd. for C₃₇H₄₀N₆O₄: 632; found: 633 (M+H)⁺. |
| Example E | 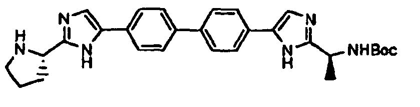 | | LCMS: Anal. Calcd. for C₂₉H₃₄N₆O₂: 498 found: 499 (M+H)⁺. |
| Example F | 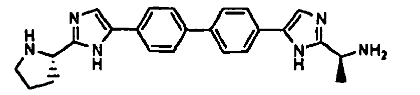 | | LCMS: Anal. Calcd. for C₂₄H₂₆N₆: 398; found: 399 (M+H)⁺. |
| Example R26 | 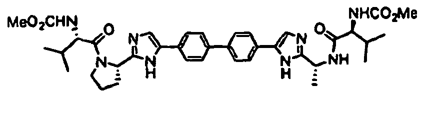 | | LCMS: Anal. Calcd. for C₃₈H₄₈N₈O₆: 712; found: 713 (M+H)⁺. |
| Example R27 | 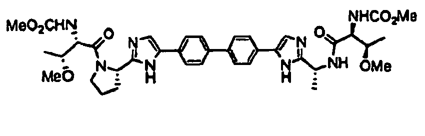 | | LCMS: Anal. Calcd. for C₃₈F₄₈N₈O₈: 744; found: 745 (M+H)⁺. |
| Example R28 | 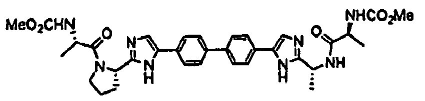 | | LCMS: Anal. Calcd. for C₃₄H₄₀N₈O₆: 756; found: 757 (M+H)⁺. |

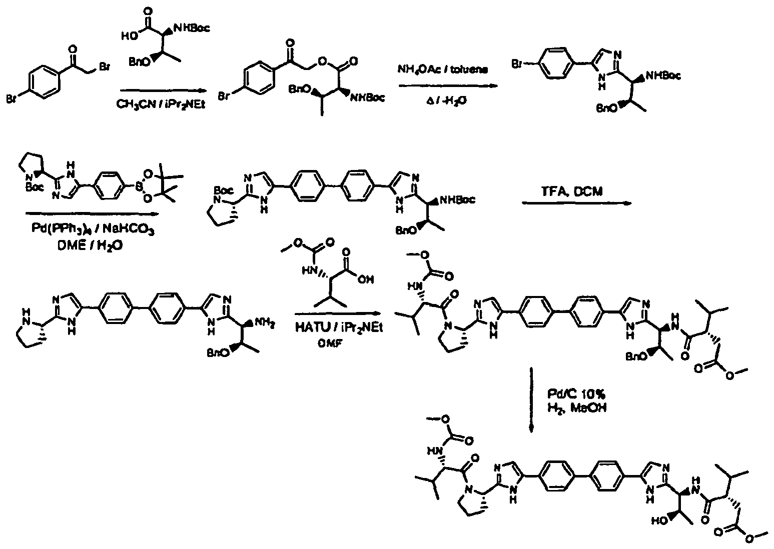

### Example R29.

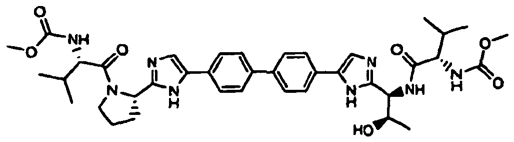

A single neck 50 mL flask equipped with a magnetic stirrer was charged with a compound from, Example (150 mg, 180 mmol) in Methanol (5 mL). To this solution was added a slurry of Palladium on carbon 10% (100 mg) in Methanol (2mL). The mixture was purged with 60 psi of Hydrogen. The mixture was shaked on a Parr overnight at room temperature. The contain of the reactor was analyzed by LC-MS and showed a 20% completion. A slurry of Palladium Hydroxide on carbon 20 % (100 mg) in Acetic acid (2 mL) was added. The mixture was purged with hydrogen and shake on a Parr shaker with 60 psi of Hydrogen over the weekend. The reaction was filtered through a pad of Celite and the filtrate was evaporated in vavuo. The residue was purified by prep HPLC (CH₃CN-H₂O-TFA) to give the compound (Example R29) 32 mg 23 % yield as a white solid. LCMS: Anal. Calcd. for C₃₉H₅₀N₈O₇: 742; found: 743 (M+H)⁺

| *Example* | *Structure* | *Analytical data* |
|---|---|---|
| Example G | 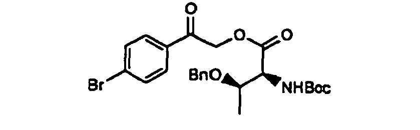 | LCMS: Anal. Calcd. for C₂₄H₂₈BrNO₆:506 found: No ionization (M+H)⁺. |
| Example H | 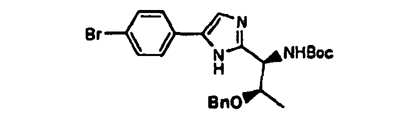 | LCMS: Anal. Calcd. for C₂₄H₂₈BrN₃O₃: 486 found: 487 (M+H)⁺. |
| Example I | 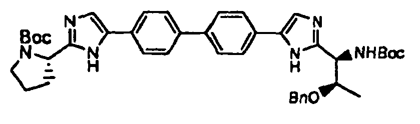 | LCMS: Anal. Calcd. for C₄₂H₅₀BN₆O₅: 718 found: 719 (M+H)⁺. |
| Example J | 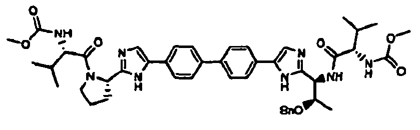 | LCMS: Anal. Calcd. for C₄₆H₅₆N₈O₇: 832; found: 833 (M+H)⁺. |
| Example R29 | 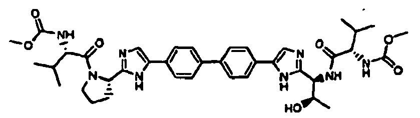 | LCMS: Anal. Calcd. for C₄₆H₅₆N₈O₇: 742 found: 743 (M+H)⁺. |

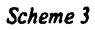

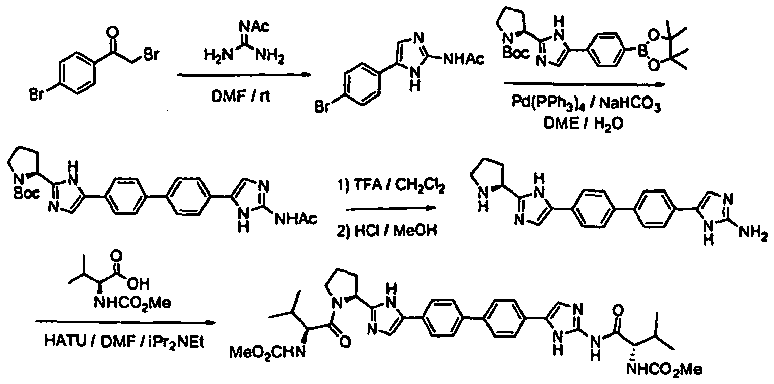

### Example R30. N-(5-(4-bromophenyl)-1H-imidazol-2-yl)acetamide.

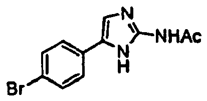

A mixture of 2-bromo-1-(4-bromophenyl)ethanone (2.00 g, 7.20 mmol) and acetylguanidine (2.18 g, 21.6 mmol) in DMF (40 mL) was stirred at rt for 48 h. The solution was concentrated to dryness in vacuo and the resulting brown solid was of sufficient purity for use in the next step. LCMS: Anal. Calcd. for C₁₁H₁₀BrN₃O: 279, 281; found: 280, 282 (M+H)⁺.

### Example R31. (S)-tert-butyl 2-(5-(4'-(2-acetamido-1H-imidazol-5-yl)biphenyl-4-yl)-1H-imidazol-2-yl)pyrrolidine-1-carboxylate

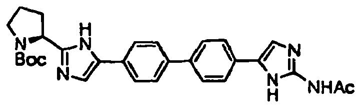

A mixture of N-(5-(4-bromophenyl)-1H-imidazol-2-yl)acetamide (0.604 g, 2.16 mmol), (S)-tert-butyl 2-(5-(4-(4,4,5,5-tetramethyl-1,3,2-dioxaborolan-2-yl)phenyl)-1H-imidazol-2-yl)pyrrolidine-1-carboxylate (0.950 g, 2.16 mmol), NaHCO₃ (0.636 g, 7.57 mmol) in DME (20 mL) and H₂O (5 mL) was degassed by evacuating and back-filling with N₂ (X3). To this solution Pd(PPh₃)₄ (0.125 g, 0.11 mmol) was added and the suspension heated at 125°C for 24h. The mixture was evaporated to dryness in vacuo and the residue was purified by column chromatography (Biotage) eluting with a gradient of 0 to 10% MeOH in CH₂Cl₂. The material was then purified by prep HPLC (CH₃CN-H₂O-NH₄OAc) to afford the title compound as a colorless solid. 7.76 (app dd, J = 8.4, 2.2 Hz, 4H), 7.69 (app d, J = 8.4 Hz, 2H), 7.66 (app d, J = 8.4 Hz, 2H), 7.45 (s, 1H), 7.23 (s, 1H), 3.66 - 3.72 (m, 1H), 3.48 - 3.53 (s, 1H), 2.34 - 2.46 (m, 1H), 2.18 (s, 3H), 1.96 - 2.10 (m, 3H), 1.46, 1.25 (s, 9H, rotamers, 1:2 ratio). LCMS: Anal. Calcd. for C₂₉H₃₂N₆O₃: 512; found: 513, 282 (M+H)⁺.

### Example R32. (S)-5-(4'-(2-(pyrrolidin-2-yl)-1H-imidazol-5-yl)biphenyl-4-yl)-1H-imidazol-2-amine.

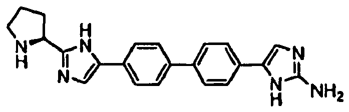

Step 1. A solution of (S)-tert-butyl 2-(5-(4'-(2-acetamido-1H-imidazol-5-yl)biphenyl-4-yl)-1H-imidazol-2-yl)pyrrolidine-1-carboxylate (55.0 mg, 0.11 mmol) in CH₂Cl₂ (5 mL) and TFA (1 mL) was allowed to stir at rt for 3 h. The reaction mixture was concentrated in vacuo to give the TFA salt of the title compound (55.0 mg) which was carried directly to the next step. LCMS: Anal. Calcd. for C₂₄H₂₄N₆O: 412; found: 413 (M+H)⁺.

Step 2. The brown oil from Step 1 was suspended in 25% conc. HCl in MeOH (10 mL) and heated at reflux for 3h. The volatiles were removed under reduced pressure and the residue was purified by prep HPLC (CH₃CN-H₂O-TFA) to give the TFA salt of the title compound (20 mg, 42%). LCMS: Anal. Calcd. for C₂₂H₂₂N₆: 370; found: 371 (M+H)⁺.

### Example R33. (S)-2-methoxycarbonylamino-N-(5-(4'-(2-((S)-1-((S)-2-methoxycarbonylamino-3-methylbutanoyl)pyrrolidin-2-yl)-1H-imidazol-5-yl)biphenyl-4-yl)-1H-imidazol-2-yl)-3-methylbutanamide

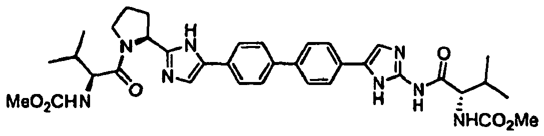

To a solution of (S)-5-(4'-(2-(pyrrolidin-2-yl)-1H-imidazol-5-yl)biphenyl-4-yl)-1H-imidazol-2-amine trifluoroacetic acid salt (20 mg, 0.04 mmol), (S)-2-(methoxycarbonylamino)-3-methylbutanoic acid (25 mg, 0.14 mmol) and iPr₂NEt (0.09 mL, 0.53 mmol) in DMF (5 mL) was added HATU (54 mg, 0.14 mmol). The solution was allowed to stir for 3h and then poured into H₂O. The mixture was concentrated to dryness in vacuo and the residue was purified by prep HPLC (CH₃CN-H₂O-TFA) affording the title compound and recovered starting material. The recovered starting material was resubjected to the reaction conditions and purified as above. The two lots of product were then purified by prep HPLC (CH₃CN-H₂O-NH₄OAc) to give the title compound as a tan solid (18 mg, 76%).
¹HNMR (400 MHz, CD₃OD) δ 7.80 - 7.88 (m, 9H), 7.56 (s, 1H), 5.24 (app t, J = 7.6 Hz, 1H), 4.23 (d, J = 7.1 Hz, 1H), 4.18 (d, J = 6.6 Hz, 1H), 4.08 - 4.13 (m, 1H), 3.83 - 3.90 (m, 1H), 3.68 (s, 3H), 3.65 (s, 3H), 2.54 - 2.63 (m, 1H), 2.17 - 2.30 (m, 4H), 2.01- 2.09 (m, 1H). LCMS: Anal. Calcd. for C₃₆H₄₄N₈O₆: 684; found: 685 (M+H)⁺.

### BIOLOGICAL ACTIVITY

An HCV Replion assay was utilized in the present disclosure, and was prepared, conducted and validated as described in commonly owned PCT/US2006/022197 and in O'Boyle et al. Antimicrob Agents Chemother. 2005 Apr;49(4):1346-53.

HCV 1b-377-neo replicon cells were used to test the currently described compound series as well as cells resistant to compound A due to a Y2065H mutation in NS5A (described in application PCT/US2006/022197). The compounds tested were determined to have more than 10-fold less inhibitory activity on cells resistant to compound A than wild-type cells indicating a related mechanism of action between the two compound series. Thus, the compounds of the present disclosure can be effective to inhibit the function of the HCV NS5A protein and are understood to be as effective in combinations as previously described in application PCT/US2006/022197 and commonly owned WO/04014852. Further, the compounds of the present disclosure can be effective against the HCV 1b genotype. It should also be understood that the compounds of the present disclosure can inhibit multiple genotypes of HCV. Table 2 shows the EC50 values of representative compounds of the present disclosure against the HCV 1b genotype. In one embodiment compounds of the present disclosure are active against the 1a, 1b, 2a, 2b, 3a, 4a, and 5a genotypes. EC50 ranges against HCV 1b are as follows: A = 1-10 µM; B = 100-999 nM; C = 1-99 nM; and D = 1-999 pM.

The compounds of the present disclosure may inhibit HCV by mechanisms in addition to or other than NS5A inhibition. In one embodiment the compounds of the present disclosure inhibit HCV replicon and in another embodiment the compounds of the present disclosure inhibit NS5A.

**Table 2**

| Example Range | |
|---|---|
| R26 | D |
| R27 | D |
| R28 | D |
| R34 | D |
| R35 | B |
| R29 | D |
| R36 | D |
| R20 | D |
| R37 | D |
| R33 | C |

It will be evident to one skilled in the art that the present disclosure is not limited to the foregoing illustrative examples, and that it can be embodied in other specific forms without departing from the essential attributes thereof. It is therefore desired that the examples be considered in all respects as illustrative and not restrictive, reference being made to the appended claims, rather than to the foregoing examples, and all changes which come within the meaning and range of equivalency of the claims are therefore intended to be embraced therein.

The compounds of the present disclosure may inhibit HCV by mechanisms in addition to or other than NS5A inhibition. In one embodiment the compounds of the present disclosure inhibit HCV replicon and in another embodiment the compounds of the present disclosure inhibit NS5A. Compounds of the present disclosure may inhibit multiple genotypes of HCV.

## Claims

1. A compound selected from
dimethyl (4,4'-biphenyldiylbis(1H-imidazole-4,2-diyl((1S)-2-methyl-1,1-propanediyl)imino((2S)-1-oxo-1,2-propanediyl)))biscarbamate;
dimethyl (4,4'-biphenyldiylbis(1H-imidazole-4,2-diyl((1S)-2-methyl-1,1-propanediyl)imino((2S,3R)-3-methoxy-1-oxo-1,2-butanediyl)))biscarbamate;
dimethyl (4,4'-biphenyldiylbis(1H-imidazole-4,2-diyl((1S)-2-methyl-1,1-propanediyl)imino((2S)-3-methyl-1-oxo-1,2-butanediyl)))biscarbamate;
dimethyl (4,4'-biphenyldiylbis(1H-imidazole-4,2-diyl((1S)-2-methyl-1,1-propanediyl)imino((2S)-4-methoxy-1-oxo-1,2-butanediyl)))biscarbam ate;
dimethyl (4,4'-biphenyldiylbis(1H-imidazole-4,2-diyl((1R)-2-methyl-1,1-propanediyl)imino((2S)-3-methyl-1-oxo-1,2-butanediyl)))biscarbamate;
dimethyl (4,4'-biphenyldiylbis(1H-imidazole-4,2-diyl((1R)-2-methyl-1,1-propanediyl)imino((2S)-1-oxo-1,2-propanediyl)))biscarbamate;
dimethyl (4,4'-biphenyldiylbis(1H-imidazole-4,2-diyl((1R)-2-methyl-1,1-propanediyl)imino((2S)-4-methoxy-1-oxo-1,2-butanediyl)))biscarbamate;
dimethyl (4,4'-biphenyldiylbis(1H-imidazole-4,2-diyl((1R)-2-methyl-1,1-propanediyl)imino((2S,3R)-3-methoxy-1-oxo-1,2-butanediyl)))biscarbamate;
dimethyl (4,4'-biphenyldiylbis(1H-imidazole-4,2-diyl((1R)-2-methyl-1,1-propanediyl)imino((2R)-3-methyl-1-oxo-1,2-butanediyl)))biscarbamate;
dimethyl (4,4'-biphenyldiylbis(1H-imidazole-4,2-diyl((1S)-2-methyl-1,1-propanediyl)imino((2R)-3-methyl-1-oxo-1,2-butanediyl)))biscarbamate;
N²-(methoxycarbonyl)-N-((1S)-1-(4-(4'-(2-((2S)-1-(N-(methoxycarbonyl)-L-valyl)-2-pyrrolidinyl)-1H-imidazol-4-yl)-4-biphenylyl)-1H-imidazol-2-yl)-2-methylpropyl)-L-valinamide;
methyl ((1S,2R)-2-methoxy-1-(((2S)-2-(4-(4'-(2-((1S)-1-((N-(methoxycarbonyl)-O-methyl-L-threonyl)amino)-2-methylpropyl)-1H-imidazol-4-yl)-4-biphenylyl)-1H-imidazol-2-yl)-1-pyrrolidinyl)carbonyl)propyl)carbamate;
methyl ((1S)-3-methoxy-1-(((2S)-2-(4-(4'-(2-((1S)-1-((N-(methoxycarbonyl)-O-methyl-L-homoseryl)amino)-2-methylpropyl)-1H-imidazol-4-yl)-4-biphenylyl)-1H-imidazol-2-yl)-1-pyrrolidinyl)carbonyl)propyl)carbamate;
methyl ((1S)-2-((2S)-2-(4-(4'-(2-((1S)-1-((N-(methoxycarbonyl)-L-alanyl)amino)-2-methylpropyl)-1H-imidazol-4-yl)-4-biphenylyl)-1H-imidazol-2-yl)-1-pyrrolidinyl)-1-methyl-2-oxoethyl)carbamate;
N²-(methoxycarbonyl)-N-((1R)-1-(4-(4'-(2-((2S)-1-(N-(methoxycarbonyl)-L-valyl)-2-pyrrolidinyl)-1H-imidazol-4-yl)-4-biphenylyl)-1H-imidazol-2-yl)-2-methylpropyl)-L-valinamide;
methyl ((1S,2R)-2-methoxy-1-(((2S)-2-(4-(4'-(2-((1R)-1-((N-(methoxycarbonyl)-O-methyl-L-threonyl)amino)-2-methylpropyl)-1H-imidazol-4-yl)-4-biphenylyl)-1H-imidazol-2-yl)-1-pyrrolidinyl)carbonyl)propyl)carbamate;
methyl ((1S)-3-methoxy-1-(((2S)-2-(4-(4'-(2-((1R)-1-((N-(methoxycarbonyl)-O-methyl-L-homoseryl)amino)-2-methylpropyl)-1H-imidazol-4-yl)-4-biphenylyl)-1H-imidazol-2-yl)-1-pyrrolidinyl)carbonyl)propyl)carbamate; and
methyl ((1S)-2-((2S)-2-(4-(4'-(2-((1R)-1-((N-(methoxycarbonyl)-L-alanyl)amino)-2-methylpropyl)-1H-imidazol-4-yl)-4-biphenylyl)-1H-imidazol-2-yl)-1-pyrrolidinyl)-1-methyl-2-oxoethyl)carbamate;
or a pharmaceutically acceptable salt thereof.

2. A compound selected from 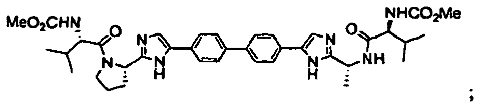 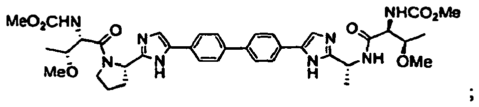 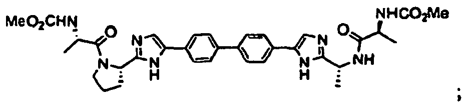 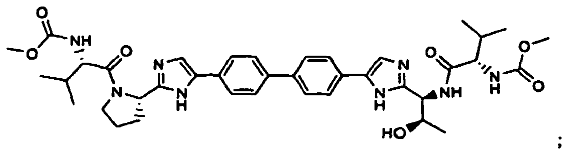 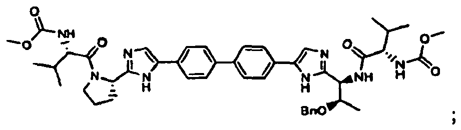 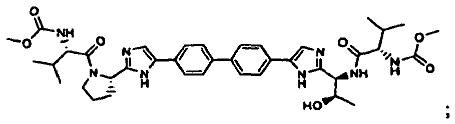 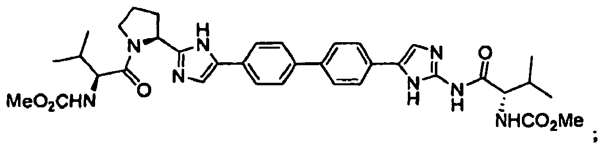 or a pharmaceutically acceptable salt thereof.

3. A composition comprising a compound of claims 1 or 2, or a pharmaceutically acceptable salt thereof, and a pharmaceutically acceptable carrier.

4. The composition of claim 3 further comprising one or two additional compounds having anti-HCV activity.

5. The composition of claim 4 wherein at least one of the additional compounds is an interferon or a ribavirin.

6. The composition of claim 5 wherein the interferon is selected from interferon alpha 2B, pegylated interferon alpha, consensus interferon, interferon alpha 2A, and lymphoblastoid interferon tau.

7. The composition of claim 4 wherein at least one of the additional compounds is selected from interleukin 2, interleukin 6, interleukin 12, a compound that enhances the development of a type 1 helper T cell response, interfering RNA, anti-sense RNA, Imiquimod, ribavirin, an inosine 5'-monophospate dehydrogenase inhibitor, amantadine, and rimantadine.

8. The composition of claim 4 wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection.

9. A compound of claims 1 or 2, or a pharmaceutically acceptable salt thereof for use in treating an HCV infection in a patient.

10. The compound for use according to claim 9 further comprising using one or two additional compounds having anti-HCV activity prior to, after or simultaneously with the compound of claims 1 or 2, or a pharmaceutically acceptable salt thereof.

11. The compound for use according to claim 10 wherein at least one of the additional compounds is an interferon or a ribavirin.

12. The compound for use according to claim 11 wherein the interferon is selected from interferon alpha 2B, pegylated interferon alpha, consensus interferon, interferon alpha 2A, and lymphoblastoid interferon tau.

13. The compound for use according to claim 10 wherein at least one of the additional compounds is selected from interleukin 2, interleukin 6, interleukin 12, a compound that enhances the development of a type I helper T cell response, interfering RNA, anti-sense RNA, Imiquimod, ribavirin, an inosine 5'-monophospate dehydrogenase inhibitor, amantadine, and rimantadine.

14. The compound for use according to claim 10 wherein at least one of the additional compounds is effective to inhibit the function of a target selected from HCV metalloprotease, HCV serine protease, HCV polymerase, HCV helicase, HCV NS4B portein, HCV entry, HCV assembly, HCV egress, HCV NS5A protein, and IMPDH for the treatment of an HCV infection.

## Patentansprüche

1. Verbindung ausgewählt unter
Dimethyl(4,4'-biphenyldiylbis(1H-imidazol-4,2-diyl((1S)-2-methyl-1,1-propandiyl)imino((2 S)-1-oxo-1,2-propandiyl)))biscarbamat;
Dimethyl(4,4'-biphenyldiylbis(1H-imidazol-4,2-diyl((1S)-2-methyl-1,1-propandiyl)imino((2S,3R)-3-methoxy-1-oxo-1,2-butandiyl)))biscarbamat;
Dimethyl(4,4'-biphenyldiylbis(1H-imidazol-4,2-diyl((1S)-2-methyl-1,1-propandiyl)imino((2S)-3-methyl-1-oxo-1,2-butandiyl)))biscarbamat;
Dimethyl(4,4'-biphenyldiylbis(1H-imidazol-4,2-diyl((1S)-2-methyl-1,1-propandiyl)imino((2S)-4-methoxy-1-oxo-1,2-butandiyl)))biscarbamat;
Dimethyl(4,4'-biphenyldiylbis(1H-imidazol-4,2-diyl((1R)-2-methyl-1,1-propandiyl)imino((2S)-3-methyl-1-oxo-1,2-butandiyl)))biscarbamat;
Dimethyl(4,4'-biphenyldiylbis(1H-imidazol-4,2-diyl((1R)-2-methyl-1,1-propandiyl)imino((2S)-1-oxo-1,2-propandiyl)))biscarbamat;
Dimethyl(4,4'-biphenyldiylbis(1H-imidazol-4,2-diyl((1R)-2-methyl-1,1-propandiyl)imino((2S)-4-methoxy-1-oxo-1,2-butandiyl)))biscarbamat;
Dimethyl(4,4'-biphenyldiylbis(1H-imidazol-4,2-diyl((1R)-2-methyl-1,1-propandiyl)imino((2S,3R)-3-methoxy-1-oxo-1,2-butandiyl)))biscarbamat;
Dimethyl(4,4'-biphenyldiylbis(1H-imidazol-4,2-diyl((1R)-2-methyl-1,1-propandiyl)imino((2R)-3-methyl-oxo-1,2-butandiyl)))biscarbamat;
Dimethyl(4,4'-biphenyldiylbis(1H-imidazol-4,2-diyl((1S)-2-methyl-1,1-propandiyl)imino((2R)-3-methyl-1-oxo-1,2-butandiyl)))biscarbamat;
N²-(Methoxycarbonyl)-N-((1S)-1-(4-(4'-(2-((2S)-1-(N-(methoxycarbonyl)-L-valyl)-2-pyrrolidinyl)-1 H-imidazol-4-yl)-4-biphenylyl)-1H-imidazol-2-yl)-2-methylpropyl)-L-valinamid;
Methyl((1S,2R)-2-methoxy-1-((2S)-2-(4-(4'-(2-((1S)-1-((N-(methoxycarbonyl)-O-methyl-L-threonyl)amino)-2-methylpropyl)-1H-imidazol-4-yl)-4-biphenylyl)-1H-imidazol-2-yl)-1-pyrrolidinyl)carbonyl)propyl)carbamat;
Methyl((1S)-3-methoxy-1-(((2S)-2-(4-(4'-(2-((1S)-1-((N-(methoxycarbonyl)-O-methyl-L-homoseryl)amino)-2-methylpropyl)-1H-imidazol-4-yl)-4-biphenylyl)-1H-imidazol-2-yl)-1-pyrrolidinyl)carbonyl)propyl)carbamat;
Methyl((1S)-2-((2S)-2-(4-(4'-(2-((1S)-1-((N-(methoxycarbonyl)-L-alanyl)amino)-2-methylpropyl)-1H-imidazol-4-yl)-4-biphenylyl)-1H-imidazol-2-yl)-1-pyrrolidinyl)-1-methyl-2-oxoethyl)carbamat;
N²-(Methoxycarbonyl)-N-((1R)-1-(4-(4'-(2-((2S)-1-(N-(methoxycarbonyl)-L-valyl)-2-pyrrodinyl)-1H-imidazol-4-yl)-4-biphenylyl)-1H-imidazol-2-yl)-2-methylpropyl)-L-valinamid;
Methyl((1S,2R)-2-methoxy-1-(((2S)-2-(4-(4'-(2-((1R)-1-((N-(methoxycarbonyl)-O-methyl-L-threonyl)amino)-2-methylpropyl)-1H-imidazol-4-yl)-4-biphenylyl)-1H-imidazol-2-yl)-1-pyrrolidinyl)carbonyl)propyl)carbamat;
Methyl((1 S)-3-methoxy-1-(((2S)-2-(4-(4'-(2-((1R)-1-((N-(methoxycarbonyl)-O-methyl-L-homoseryl)amino)-2-methylpropyl)-1H-imidazol-4-yl)-4-biphenylyl)-1H-imidazol-2-yl)-1-pyrrolidinyl)carbonyl)propyl)carbamat; und
Methyl((1S)-2-((2S)-2-(4-(4'-(2-((1R)-1-((N-(methoxycarbonyl)-L-alanyl)amino)-2-methylpropyl)-1H-imidazol-4-yl)-4-biphenylyl)-1H-imidazol-2-yl)-1-pyrrolidinyl)-1-methyl-2-oxoethyl)carbamat;
oder ein pharmazeutisch akzeptables Salz davon.

2. Verbindung ausgewählt unter 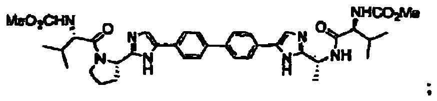 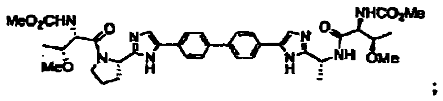 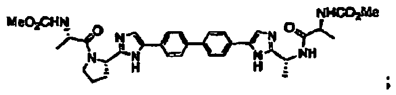 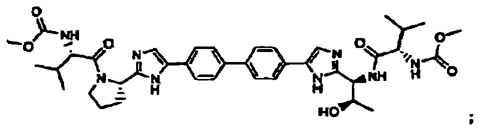 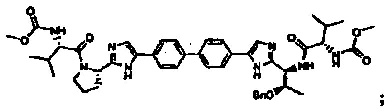 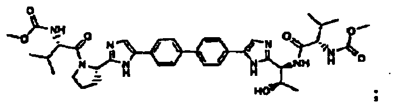 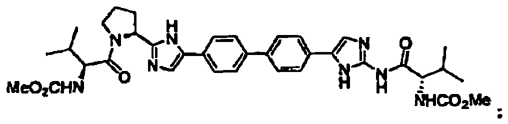 oder ein pharmazeutisch akzeptables Salz davon.

3. Zusammensetzung umfassend eine Verbindung nach den Ansprüchen 1 oder 2 oder ein pharmazeutisch akzeptables Salz davon und ein pharmazeutisch akzeptabler Träger.

4. Zusammensetzung nach Anspruch 3, des Weiteren eine oder zwei zusätzliche Verbindungen umfassend, die Anti-HCV-Aktivität aufweisen.

5. Zusammensetzung nach Anspruch 4, wobei mindestens eine der zusätzlichen Verbindungen ein Interferon oder ein Ribavirin ist.

6. Zusammensetzung nach Anspruch 5, wobei das Interferon unter Interferon-Alpha 2B, pegyliertem Interferon-Alpha, Konsensus-Interferon, Interferon-Alpha 2A und lymphoblastoidem Tau-Interferon ausgewählt wird.

7. Zusammensetzung nach Anspruch 4, wobei mindestens eine der zusätzlichen Verbindungen unter Interleukin 2, Interleukin 6, Interleukin 12, einer Verbindung, die die Entwicklung einer Typ 1-T-Helferzellenantwort verbessert, interferierender RNA, Antisense-RNA, Imiquimod, Ribavirin, einem Inosin-5'-monophosphat-Dehydrogenaseinhibitor, Amantadin und Rimantadin ausgewählt wird.

8. Zusammensetzung nach Anspruch 4, wobei mindestens eine der zusätzlichen Verbindungen wirksam ist, die Funktion eines Targets, ausgewählt unter HCV-Metalloprotease, HCV-Serinprotease, HCV-Polymerase, HCV-Helicase, HCV NS4B-Protein, HCV-Eingang, HCV-Ansammlung, HVC-Abgang, HCV NS5A-Protein und IMPDH zu hemmen, zur Behandlung einer HCV-Infektion.

9. Verbindung nach Anspruch 1 oder 2 oder ein pharmazeutisch akzeptables Salz davon zur Verwendung zur Behandlung einer HCV-Infektion bei einem Patienten.

10. Verbindung zur Verwendung nach Anspruch 9, des Weiteren umfassend das Verwenden von einer oder zwei zusätzlichen Verbindungen, die Anti-HCV-Aktivität aufweisen, vor, nach oder gleichzeitig mit der Verbindung nach Anspruch 1 oder 2 oder eines pharmazeutisch akzeptablen Salzes davon.

11. Verbindung zur Verwendung nach Anspruch 10, wobei mindestens eine der zusätzlichen Verbindungen ein Interferon oder ein Ribavirin ist.

12. Verbindung zur Verwendung nach Anspruch 11, wobei das Interferon unter Interferon-Alpha 2B, pegyliertem Interferon-Alpha, Konsensus-Interferon, Interferon-Alpha 2A und lymphoblastoidem Tau-Interferon ausgewählt wird.

13. Verbindung zur Verwendung nach Anspruch 10, wobei mindestens eine der zusätzlichen Verbindungen unter Interleukin 2, Interleukin 6, Interleukin 12, einer Verbindung, die die Entwicklung einer Typ 1-T-Helferzellenantwort verbessert, interferierender RNA, Antisense-RNA, Imiquimod, Ribavirin, einem Inosin-5'-monophosphat-Dehydrogenaseinhibitor, Amantadin und Rimantadin ausgewählt wird.

14. Verbindung zur Verwendung nach Anspruch 10, wobei mindestens eine der zusätzlichen Verbindungen wirksam ist, die Funktion eines Targets, ausgewählt unter HCV-Metalloprotease, HCV-Serinprotease, HCV-Polymerase, HCV-Helicase, HCV NS4B-Protein, HCV-Eingang, HCV-Ansammlung, HVC-Abgang, HCV NS5A-Protein und IMPDH zu hemmen, zur Behandlung einer HCV-Infektion.

## Revendications

1. Composé sélectionné parmi
biscarbamate de diméthyl-(4,4'-biphényldiylbis(1H-imidazole-4,2-diyl((1S)-2-méthyl-1,1-propanediyl)imino((2S)-1-oxo-1,2-propanediyle)));
biscarbamate de diméthyl-(4,4'-biphényldiylbis(1H-imidazole-4,2-diyl((1S)-2-méthyl-1,1-propanediyl)imino((2S,3R)-3-méthoxy-1-oxo-1,2-butanediyle)));
biscarbamate de diméthyl-(4,4'-biphényldiylbis(1H-imidazole-4,2-diyl((1S)-2-méthyl-1,1-propanediyl)imino((2S)-3-méthyl-1-oxo-1,2-butanediyle)));
biscarbamate de diméthyl-(4,4'-biphényldiylbis(1H-imidazole-4,2-diyl((1S)-2-méthyl-1,1-propanediyl)imino((2S)-4-méthoxy-1-oxo-1,2-butanediyle)));
biscarbamate de diméthyl-(4,4'-biphényldiylbis(1H-imidazole-4,2-diyl((1R)-2-méthyl-1,1-propanediyl)imino((2S)-3-méthyl-1-oxo-1,2-butanediyle)));
biscarbamate de diméthyl-(4,4'-biphényldiylbis(1H-imidazole-4,2-diyl((1R)-2-méthyl-1,1-propanediyl)imino((2S)-1-oxo-1,2-propanediyle)));
biscarbamate de diméthyl-(4,4'-biphényldiylbis(1H-imidazole-4,2-diyl((1R)-2-méthyl-1,1-propanediyl)imino((2S)-4-méthoxy-1-oxo-1,2-butanediyle)));
biscarbamate de diméthyl-(4,4'-biphényldiylbis(1H-imidazole-4,2-diyl((1R)-2-méthyl-1,1-propanediyl)imino((2S,3R)-3-méthoxy-1-oxo-1,2-butanediyle)));
biscarbamate de diméthyl-(4,4'-biphényldiylbis(1H-imidazole-4,2-diyl((1R)-2-méthyl-1,1-propanediyl)imino((2R)-3-méthyl-1-oxo-1,2-butanediyle)));
biscarbamate de diméthyl-(4,4'-biphényldiylbis(1H-imidazole-4,2-diyl((1S)-2-méthyl-1,1-propanediyl)imino((2R)-3-méthyl-1-oxo-1,2-butanediyle)));
N²-(méthoxycarbonyl)-N-((1S)-1-(4-(4'-(2-((2S)-1-(N-(méthoxycarbonyl)-L-valyl)-2-pyrrolidinyl)-1H-imidazol-4-yl)-4-biphénylyl)-1H-imidazol-2-yl)-2-méthylpropyl)-L-valinamide;
carbamate de méthyl-((1S,2R)-2-méthoxy-1-(((25)-2-(4-(4'-(2-((1S)-1-(N-méthoxycarbonyl)-O-méthyl-L-thréonyl)amino)-2-méthylpropyl)-1H-imidazol-4-yl)-4-biphénylyl)-1H-imidazol-2-yl)-1-pyrrolidinyl)carbonyl)propyle);
carbamate de méthyl-((1S)-3-méthoxy-1-(((2S)-2-(4-(4'-(2-((1S)-1-(N-méthoxycarbonyl)-O-méthyl-L-homoséryl)amino)-2-méthylpropyl)-1H-imidazol-4-yl)-4-biphénylyl)-1H-imidazol-2-yl)-1-pyrrolidinyl)carbonyl)propyle);
carbamate de méthyl-((1S)-2-((2S)-2-(4-(4'-(2-((1S)-1-(N-méthoxycarbonyl)-L-alanyl)amino)-2-méthylpropyl)-1H-imidazol-4-yl)-4-biphénylyl)-1H-imidazol-2-yl)-1-pyrrolidinyl)-1-méthyl-2-oxoéthyle);
N²-(méthoxycarbonyl)-N-((1R)-1-(4-(4'-(2-((2S)-1-(N-(méthoxycarbonyl)-L-valyl)-2-pyrrolidinyl)-1H-imidazol-4-yl)-4-biphénylyl)-1H-imidazol-2-yl)-2-méthylpropyl)-L-valinamide;
carbamate de methyl-((1S,2R)-2-méthoxy-1-(((2S)-2-(4-(4'-(2-((1R)-1-(N-méthoxycarbonyl)-O-méthyl-L-thréonyl)amino)-2-méthylpropyl)-1H-imidazol-4-yl)-4-biphénylyl)-1H-imidazol-2-yl)-1-pyrrolidinyl)carbonyl)propyle);
carbamate de methyl-((1S)-3-méthoxy-1-(((2S)-2-(4-(4'-(2-((1R)-1-(N-méthoxycarbonyl)-O-méthyl-L-homoséryl)amino)-2-méthylpropyl)-1H-imidazol-4-yl)-4-biphénylyl)-1H-imidazol-2-yl)-1-pyrrolidinyl)carbonyl)propyle); et
carbamate de méthyl-((1S)-2-((2S)-2-(4-(4'-(2-((1R)-1-(N-méthoxycarbonyl)-L-alanyl)amino)-2-méthylpropyl)-1H-imidazol-4-yl)-4-biphénylyl)-1H-imidazol-2-yl)-1-pyrrolidinyl)-1-méthyl-2-oxoéthyle);
ou un sel pharmaceutiquement acceptable de ceux-ci.

2. Composé sélectionné parmi 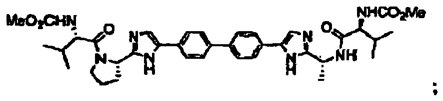 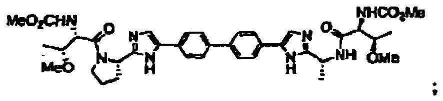 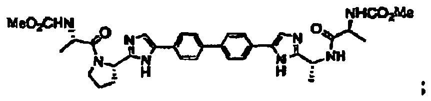 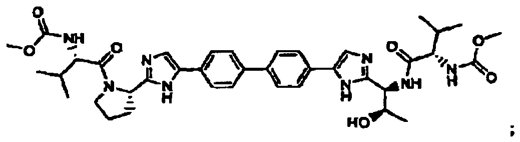 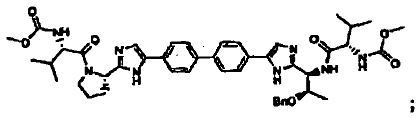 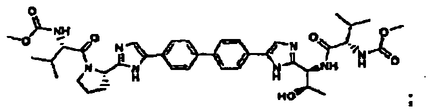 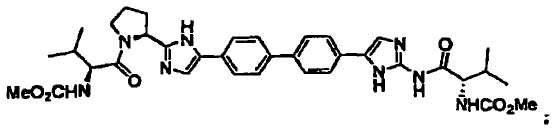 ou un sel pharmaceutiquement acceptable de ceux-ci.

3. Composition comprenant un composé selon les revendications 1 ou 2 ou un sel pharmaceutiquement acceptable de ceux-ci et un véhicule pharmaceutiquement acceptable.

4. Composition selon la revendication 3, comprenant en outre un ou deux composés supplémentaires ayant une activité anti-VHC.

5. Composition selon la revendication 4, dans laquelle au moins l'un des composés supplémentaires est un interféron ou une ribavirine.

6. Composition selon la revendication 5, dans laquelle l'interféron est sélectionné parmi l'interféron alpha 2B, l'interféron alpha pégylé, l'interféron consensus, l'interféron alpha 2A et l'interféron tau lymphoblastoïde.

7. Composition selon la revendication 4, dans laquelle au moins l'un des composés supplémentaires est sélectionné parmi l'interleukine 2, l'interleukine 6, l'interleukine 12, un composé qui augmente le développement d'une réponse des cellules T auxiliaires de type 1, un ARN interférent, un ARN anti-sens, l'imiquimod, la ribavirine, un inhibiteur d'inosine 5'-monophosphate déshydrogénase, l'amantadine et la rimantadine.

8. Composition selon la revendication 4, dans laquelle au moins l'un des composés supplémentaires est efficace pour inhiber la fonction d'une cible sélectionnée parmi la métalloprotéase du VHC, la sérine protéase du VHC, la polymérase du VHC, l'hélicase du VHC, la protéine NS4B du VHC, l'entrée du VHC, l'assemblage du VHC, la sortie du VHC, la protéine NS5A du VHC et l'IMPDH, dans le traitement d'une infection par le VHC.

9. Composé selon les revendications 1 ou 2 ou un sel pharmaceutiquement acceptable de ceux-ci, à utiliser dans le traitement d'une infection par le VHC chez un patient.

10. Composé à utiliser selon la revendication 9, comprenant en outre utiliser un ou deux composés supplémentaires ayant une activité anti-VHC, avant, après ou simultanément avec le composé selon les revendications 1 ou 2 ou un sel pharmaceutiquement acceptable de ceux-ci.

11. Composé à utiliser selon la revendication 10, où au moins l'un des composés supplémentaires est un interféron ou une ribavirine.

12. Composé à utiliser selon la revendication 11, où l'interféron est sélectionné parmi l'interféron alpha 2B, l'interféron alpha pégylé, l'interféron consensus, l'interféron alpha 2A et l'interféron tau lymphoblastoïde.

13. Composé à utiliser selon la revendication 10, où au moins l'un des composés supplémentaires est sélectionné parmi l'interleukine 2, l'interleukine 6, l'interleukine 12, un composé au augmente le développement d'une réponse des cellules T auxiliaires de type 1, un ARN interférent, un ARN anti-sens, l'imiquimod, la ribavirine, un inhibiteur d'inosine 5'-monophosphate déshydrogénase, l'amantadine et la rimantadine.

14. Composé à utiliser selon la revendication 10, où au moins l'un des composés supplémentaires est efficace pour inhiber la fonction d'une cible sélectionnée parmi la métalloprotéase du VHC, la sérine protéase du VHC, la polymérase du VHC, l'hélicase du VHC, la protéine NS4B du VHC, l'entrée du VHC, l'assemblage du VHC, la sortie du VHC, la protéine NS5A du VHC et l'IMPDH, dans le traitement d'une infection par le VHC.
